# EUROPEAN PATENT SPECIFICATION

(11) **EP 1 343 876 B1**
(45) Date of publication and mention of the grant of the patent: **14.02.2007**
(21) Application number: 01990050.5
(22) Date of filing: 11.12.2001
(51) Int. Cl.: C12N 9/12, A61K 38/04, C07K 7/00, A61K 38/45, G01N 33/68, A61P 35/00, A61P 37/00

(54) **MODULATORS OF ACTIVITY OF G-PROTEIN-COUPLED RECEPTOR KINASES**
MODULATOREN DER AKTIVITÄT VON G-PROTEIN GEKOPPELTEN REZEPTOR KINASEN
MODULATEURS DE L'ACTIVITE DE KINASES DE RECEPTEURS COUPLES A LA PROTEINE G

(30) Priority: 11.12.2000 US 735274
(43) Date of publication of application: 17.09.2003
(73) Proprietor: CHILDREN'S MEDICAL CENTER CORPORATION, Boston Massachusetts 02115 (US); Yissum Research and Development, 91042 Jerusalem (IL)
(72) Inventor: BEN-SASSON, Shmuel, 96555 Jerusalem (IL)
(74) Representative: Lönnqvist, Gunnel Solveig Kristina
(86) International application number: PCT/US2001/047508
(87) International publication number: WO 2002/047711

(56) References cited:
- WO-A-00/18895
- WO-A-00/73469
- WO-A-98/53050
- WO-A-98/53051

## Description

The present invention is related to GRK-assosciated signal transduction (GAST) inhibitors, defined in the claims, particularly to the compound K024H107 (SEQ ID NO.: 10). Said inhibitors are useful as medicines particularly for preparing medicines for treating obesity, dyslipidemia, cholesterolemia, coagulation disorders, syndrome X, diabetes, hypertension or ateriosclerosis, particularly diabetic associated phenomena such as diabetes type II, diabetic-associated obesity, diabetic related hypertension or diabetic associated dyslipidemia.

Serine/threonine kinases are members of the eukaryotic protein kinase superfamily. Enzymes of this class specifically phosphorylate serine or threonine residues of intracellular proteins and are important in mediating signal transduction in multicellular organisms. Many serine/threonine kinases occur as intracellular proteins which take part in signal transduction within the cell, including signal transduction to the nucleus and the activation of other proteins.

As such, phosphorylation of serine or threonine by serine/threonine kinases is an important mechanism for regulating intracellular events in response to environmental changes. A wide variety of cellular events are regulated by serine/threonine kinases. A few examples include the ability of cells to enter and/or complete mitosis, cellular proliferation, cellular differentiation, the control of fat metabolism, immune responses, inflammatory responses and the control of glycogen metabolism.

An important superfamily of cell membrane receptors is the group known as G-protein coupled receptors (GPCR), known also as seven trans-membrane receptors (7TM). This superfamily of receptors is involved in the transmission of signals that originate from low molecular weight ligands such as adrenaline or from peptide ligands such as chemokines and a variety of hormones such as melanocyte stimulating hormone (MSH).

Numerous studies have shown that intracellular protein kinases which specifically interact with various members of the 7TM receptors are able to desensitize them and thereby decrease or eliminate the signal transmission effected by 7TM. These protein kinases are known as G-protein-coupled receptor kinases (GRKs), which are serine/threonine kinases. So far, six of these kinases have been discovered (GRK1-6). Some of the GRKs are restricted to a small number of tissues (e.g., GRK1), while GRK2 and GRK 3, known also as β-ARK1 and β-ARK2 are ubiquitously expressed. A comprehensive review is provided, for example, by M. Bunemann and M.M. Hosey, "G-Protein Coupled Receptor Kinases as Modulators of G-Protein Signalling," *J of Physiology,* **Vol. 517(1):**5-23 (1999).

Syndrome X is a term coined in 1988 by Stanford University Endocrinologist Dr. Gerald Risson, that describes a group of symptoms including: high blood pressure, abdominal obesity, insulin resistance, high levels of triglycerides and low levels of HDL, low levels of anti-oxidant vitamins and DHEA, high cortisone levels, as well as depression. Some experts estimate that as many as two-thirds of Americans may be suffering from syndrome X, although it may be effectively hidden for years masquerading as symptoms for other conditions such as fatigue, poor mental concentration, abdominal obesity, edema, nerve damage and intense craving for sweets.

The International patent applications WO 9853050, WO 9853051, and WO 200018895 disclose protein kinase modulating peptides of the HJ loop of serine/threonine kinase, the HJ loop of serine/tyrosine kinase,, the αD region of the of a protein kinase. None of the protein kinases discussed in said patent applications are useful for preparing medicines for treating obesity, dyslipidemia, cholesterolemia, coagulation disorders, syndrome X, diabetes, hypertension or ateriosclerosis, particularly diabetic associated phenomena such as diabetes type II, diabetic-associated obesity, diabetic related hypertension or diabetic associated dyslipidemia. that the above peptides modulate the GRK associated signal transduction. These two findings led to the realization that modulation of GRK associated signal transduction can alleviate a plurality of metabolic-related disorders and diseases.

The present invention is related to the following compounds K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.: 6), K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO.: 17), K024H901 (SEQ ID NO.: 18), K024H903 (SEQ ID NO.: 19), or and SEQ ID NO: 20 to SEQ ID NO: 38. Said compounds, which are GRK-assosciated signal transduction (GAST) inhibitors, and particularly the compound K024H107 (SEQ ID NO.: 10), are useful as medicines, and particularly for preparing a medicaments for treating obesity, dyslipidemia, cholesterolemia, coagulation disorders, syndrome X, diabetes, hypertension or ateriosclerosis, particularly diabetic associated phenomena such as diabetes type II, diabetic-associated obesity, diabetic related hypertension or diabetic associated dyslipidemia.

The present invention is based on a method for preparation of a medicament for the modulation of a metabolic parameter in a subject the method comprising:
administering to a subject in need of such treatment a therapeutically effective amount of a compound comprising a sequence selected from Seq. Id. 2-38.

The term *"modulation"* refers to increase or decrease of the metabolic parameter tested, for example; decrease in blood glucose, decrease in appetite, decrease in weight, increase in melanogenesis, increase in basal metabolism etc. This term also refers to or a change in the response of the metabolic parameter to effectors, such as change in the level of blood glucose (the metabolic parameter) in response to effectors (for example, insulin administration, stress, glucose loading) as compared to control.

The term *"metabolic parameter"* refers to at least one measurable, physiological phenomena which can be indicative of the activity of a metabolic pathway. The following table will give a list of metabolic parameters that can be modulated by the method of the invention, and the disease that may be treated by modulation of the parameter.

| **Metabolic Parameter** | **Disease** |
|---|---|
| Glucose level or insulin level | Diabetes (type I or II) |
| Plasma lipid profile (HDL level, cholesterol level, LDL level, HDL/LDL/ratio total lipid profile | Arteriosclerosis, cholesterolinemia hyerlipidemia, dislipidemia |
| Blood pressure | Hypertension |
| Abnormalities in blood coagulation/higher plasmonogen activator inhibitor Type I and fibrinogen levels | Coagulation disorders - especially associated with syndrome X |
| Albumin or protein in urine | Hyperuricemica/micro albumenia |
| Presence of cysts in ovaries | Polycystic ovarian syndrome |
| Food consumption | Obesity-(Syndrome X) |
| Change in body weight | Obesity-(Syndrome X) |
| Basal-metabolic rate | Obesity-(Syndrome X) |

Modulation of an individual's metabolism refers to an inhibition or enhancement of metabolic processes such as glucose uptake (insulin dependent or independent), lipid breakdown or synthesis, gluconeogenesis, glycogenolysis, cellular uptake of free fatty acids and triglycerides and cholesterol metabolism compared to a base line level for the individual, as known in the art.

*"Modulation of a metabolic parameter*" refers to enhanced melanogenesis, alteration of Syndrome X, correction of Type II diabetes mellitus, improvement of heart function, relief of hypertension, improved blood lipid profile and lowered propensity for obesity. Methods of determining changes in these functions and activities are well known in the art and are further described below.

The present invention further concerns the use of a compound defined in the claims for preparing a medicine for the treatment of diabetes and diabetic-associated phenomena said compound being selected from the Seq. Id. nos. 2-38.

The term *"diabetes"* in the context of the present invention concerns both diabetic mellitus Type II caused by insulin resistance and diabetic mellitus Type I caused by decrease of insulin secretion. In the latter case, there should be at least some intrinsic insulin concentration present in the subjects serum (as the compound works by GPCR pathway), either as a result of basal secretion or as a result of external administration. Thus, the compounds of the invention may be used together with insulin, thus lowering the required dosage, or decreasing the episodes of administration.

The term *"treatment of diabetic-associated phenomena"* includes: decrease in blood glucose levels, improved response (as regards control glucose blood levels) to effectors such as insulin administration, fasting or glucose loading, as well as improvement in at least one of diabetes-associated phenomena as described above including obesity, hypertension, dislipidemia and the like.

The present invention also concerns use of a compound defined in the claims comprising a sequence selected from Seq. Id. 2-38 for the preparation of a medicament for the treatment of a disease selected from: diabetes, hypertension, obesity, dislipidemia, congestive heart disease, arteriosclerosis, cholesterolinemia, coagulation disorders and syndrome X

The most favorable compounds defined in the claims are present invention from the above sequences (a) to (i), for the modulation of a metabolic parameter.

Thus the compounds of the present invention are obtained by a method including the modulation of a metabolic parameter, the method comprising:
(a) identifying peptide regions in GRK that are in positions selected from: 382-414 (HJ-loop), 271-290 (A-region), 257-265 (B4-B5 region), 240-260 (αD region);
(b) synthesizing a plurality of compounds comprising a sequence selected from:
   (b1) a sequence corresponding to at least five continuous amino acid sequences of the HJ-loop, A-region, B4-B5 or αD region;
   (b2) a variant of a sequence according to (b1) wherein up to 40% amino acids of the native sequence have been replaced with a naturally or non-naturally occurring amino acid or with a peptidomimetic organic moiety; and/or up to 40% of the amino acids have their side chains chemically modified; and/or up to 20% of the amino acids have been deleted, provided that the variant has at least 50% of the amino acids present in the sequence of (b1) un altered and that the variant maintains the biological property of the parent amino acids of(bl);
   (b3) a sequence of (b1) or (b2) wherein one or more of the amino acids has been replaced by the corresponding D-amino acid;
   (b4) a sequence of (b1), (b2) or (b3) wherein at least one peptidic backbone has been altered to a non-naturally occurring peptidic backbone;
   (b5) a sequence being the sequence of any one of (b1), (b2), (b3) or (b4) in a reverse order; and
   (b6) a combination of two or more sequences of (b1)-(b5);
(c) testing the modulation activity of the compounds of (b) in a test assay for determining the level of at least one metabolic parameter;
(d) selecting from the compounds of (c) those compounds which modulated the metabolic parameter in the test assay as compared to the modulation in the same test assay in the absence of the compound; and
(e) producing the compounds of (d) thereby obtaining compounds for the modulation of the metabolic parameter.

The amount of compounds of the invention administered to the individual will depend on the type and severity of the disease (for example, the level of hyperinsulinemia) and on the characteristics of the individual, such as general health, age, body weight, sex and tolerance to drugs as well as on the mode of administration. The skilled artisan will be able to determine appropriate dosages depending on these and other factors. Typically, a therapeutically effective amount of the compound can range from about 1 mg per day to about.1000 mg per day for an adult Preferably, the dosage ranges from about 1 mg per day to about 100 mg per day.

By a second aspect the present invention concerns the use of the GAST-inhibitors defined in the claims for preparing a medicine for the treatment of an diabetic-associated phenomena.

The term *"diabetic-associated phenomena"* refers to diabetes itself, in particular diabetes type II, as well as the directed undesirable manifestations caused by diabetes measured by determination of: glucose blood levels, diabesity (diabetic-associated obesity), diabetic related hypertension and diabetic associated dislipedemia.

Among the GAST inhibitors that can be employed are compounds defined in the claims and comprising sequences derived from GRK regions responsible for interaction with cellular components, or variants of such sequences as described above; antibodies immunoreactive with GRK; anti-sense nucleic acids that block expression of GRK; negative-dominant GRK genes which express GRK proteins with reduced or non-existent biological activity, ribozymes capable of specifically cleaving GRK-RNA and small organic molecules. Any of these inhibitors of GAST will be able to improve the diabetic-associated phenomena and thus be suitable for treatment of a disease as described above.

Preferably the GAST inhibitors defined in the claims are compounds comprising sequences derived from regions of the GRK which are responsible for interaction with other cellular components, especially with the GPCR substrate. As indicated above, it is assumed that peptides mimicking said regions, bind to the cellular components (such as the GPCR substrate of the GRK), and by this interrupt the interaction of the GRK-kinase and the substrate, leading to modulation of GAST.

More specifically, the GAST modulator is selected from (i) a compound comprising a sequence selected from: Seq. Id. no. 2-38.

Specific examples of the compounds of (i) are compounds which comprise any one of the sequences as specified in: K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.: 6), K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO.: 17), K024H901 (SEQ ID NO.: 18), and K024H903 (SEQ ID NO.: 19), or any one of SEQ ID NOS.:20 to 38.

The term *"GRK-associated signal transduction (GAST)"* refers to the level of signaling mediated by that GPCR, which the GRK2 or GRK3 phosphorylates. Typically the level can be determined by determining the level of the phosphorylation of at least one substrate in the GRK-signaling pathway, which may be a direct substrate of GRK (GPCR receptors such as β_{2/1}-adrenergic receptor, α₂-adrenergic receptor, acetylcholine receptor, opioid receptors, rhodopsin ,A_{(1,2,3)}-purinergic receptor" synuclein, Angiotensin II 1a, DᵢA-dopamine, N-formyl peptide, muscarinic receptor, platelet activating factor, thrombin etc (Bunemann *et al, J. of Physiology* (1999) 517.1,5-23), or a substrate of another kinase more downstream in the GRK- kinase signaling pathway.

The sequences defined in the claims which correspond to regions of GRK, in addition to their ability to modulate a metabolic parameter also are useful for generating antibodies that can modulate GRK-associated signal transduction, thus modulating metabolism. The sequences act as antigenic agents for producing such antibodies. These antibodies, in turn, act as inhibitors of GAST, thereby modulating metabolism.

In order to understand the invention and to see how it may be carried out in practice, a preferred embodiment will now be described, by way of non-limiting example only, with reference to the accompanying drawings, in which:

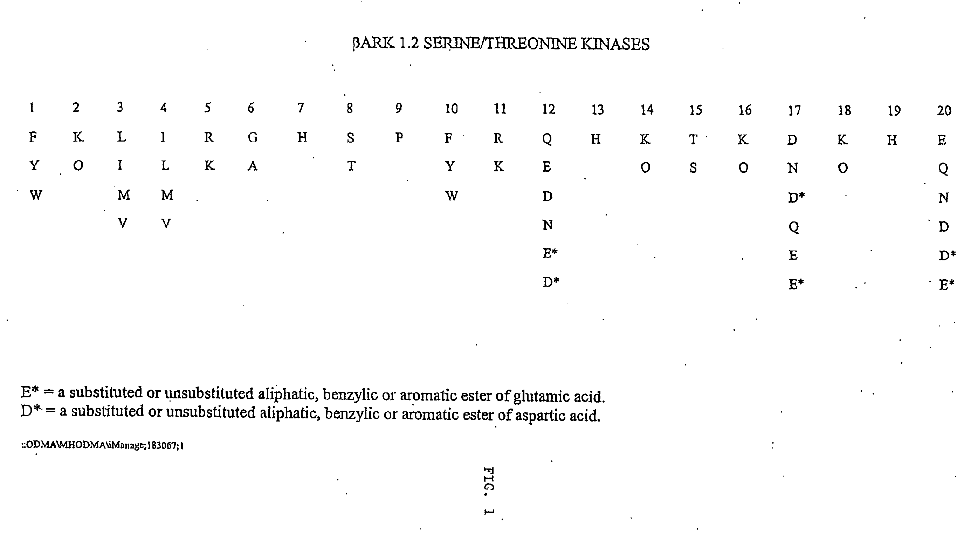

**Fig. 1** is a table illustrating the amino acid sequences of the HJ loop of GRK2 and GRK3, also referred to herein as βARK1 (or βARK1) and βARK2 (or βARK2).

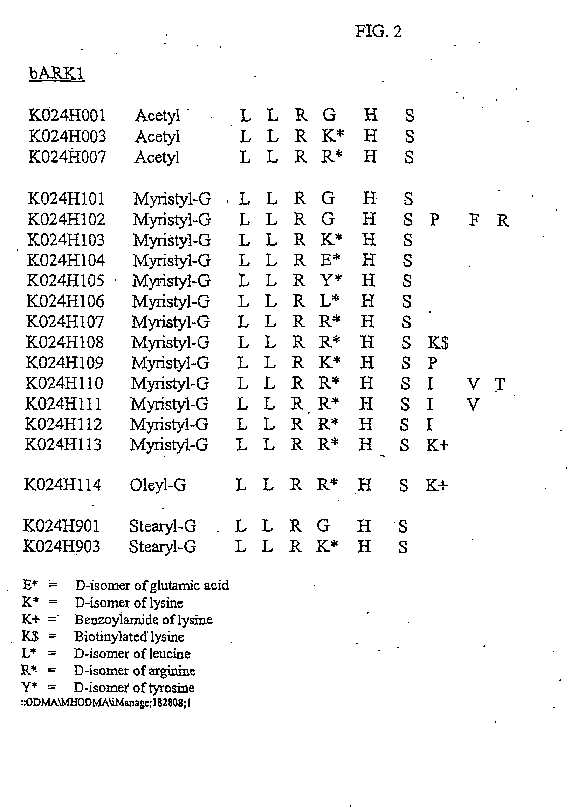

**Fig. 2** is a table illustrating the sequences of peptides K024H001 (SEQ ID NO.: 1), K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.: 6), K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO. : 17), K024H901 (SEQ ID NO.: 18), and K024H903 (SEQ ID NO.: 19).
**Fig. 3** is a graph that shows the effects of a single injection of the compound of the invention comprising a GRK-derived peptide on blood-glucose of sand rats (*psamomys obesus*)*.*
**Fig. 4** is a graph that shows blood-glucose of control untreated sand rats (*psamomys obesus*)*.*
**Figs. 5A-5G** are graphs illustrating the effects of peptides of the invention on melanogenesis by murine B16 melanoma cells.
**Fig. 6** shows the effect of the compound of the invention K024H107 on the production of cAMP, administered before or after activation by isoproteranol.
**Fig. 7A** shows the effect of compound K024H107 of the invention administered to normal mice on weight changes and Fig. 7B shows the effect of food consumption,.
**Fig. 8A** shows the effect of the compound K024H112 administered to normal mice n on the weight and Fig. 8B shows the effect on food consumption of normal mice.

It has been found in accordance with the present invention that modulation of activity of GAST influences a variety of signal-transduction pathways. For example, inhibition of a GAST, i.e. elimination of the agonist-dependent desensitizing activity caused by phosphorylation of the GPCR by the GRK, can result in a stronger or more extended signal by the relevant GPCR receptor; e.g., extending the duration of hormonal effects of, for example, adrenaline or any ligand activating the receptor. Thus, agents which modulate the activity of GAST can be used in the treatment of diseases that result from a lower bioavailability of the corresponding GPCR ligand, such as low availability of epinephrie, dopamine angiotensine or any other GPCR-ligand.

A particular intriguing situation with this invention is the systemic administration of a GAST modulator. Under such circumstances, multiple systems can be affected simultaneously. Without wishing to be bound by a particular mechanism, it is believed that, if all of the systems which control the metabolic activity of the body are tuned by the same molecular mechanism, namely GRK activity, then a systemic inhibition of GRK 2, GRK3 or its associated signal transduction pathway will have a simple phenotypic result: increase in the overall body basal metabolic rate due to elimination of GPCR desensitization and prolonging of its activity. Such a result is favorable in the condition now known as "syndrome" which is typified by the onset of type II diabetes mellitus, obesity and other conditions especially diabetic-associated phenomena. For a review of syndrome-x, see O. Timar *et al.,* "Metabolic Syndrome X: A Review, " *Can. J. Cardiol,* **16**(6): 779-789 (2000).

With this invention, inhibiting the effects of GRK-2, GRK-3 which can be thought of as a metabolic regulator, is a method of treating type 2 diabetes mellitus (DM). It appears that in specific low calorie environments, organisms including humans, have evolved a mechanism by which maximal energy metabolism is achieved by down-regulating metabolic processes. It is postulated in this invention that the mechanism for this down-regulation is phosphorylation of β-adrenergic receptors (βAR) by GRK-2 (β-adrenergic receptor kinase). The attenuated βAR leads to decreased signaling to significant metabolic processes such as glucose uptake (via insulin resistance), lipid breakdown, diabetic-associated obesity, diabetic associated hypertension etc. This enables the organism to maintain energy homeostasis despite low exogenous caloric intake.

Nutritional diabetes can be caused by a pathologic function of the interaction between βAR and GRK-2. When organisms that are maximally adapted to a low energy environment are transferred to a high-energy environment, they develop a metabolic syndrome characterized by type 2 diabetes mellitus (DM), hypertension, obesity, insulin resistance and other diabetic-associated phenomena as described above.. This is due to the surfeit of energy, which is inefficiently utilized because of the low metabolic rate. The surplus energy is converted to fat and there is a hyperglycemia due to insulin resistance in the face of high glucose levels. By decreasing the activity of GRK-2, the activity of βAR is increased and the metabolic rate is increased.

The concept of a metabolic regulator comes from an animal model of nutritional DM. *Psamomys obesus,* a desert gerbil that survives on a low energy diet, develops insulin resistance and type 2 DM when placed on a high energy diet. As shown herein, diabetes is corrected when GRK-2 activity is inhibited, thereby supporting the concept that manipulation of a metabolic rheostat is a treatment for DM.

The following information further substantiates the concept of such a metabolic rheostat: Up-regulation of GRK also causes decreased βAR in the heart which exacerbates heart failure. Inhibition of GAST by an inhibitor delivered locally to the heart may improve its function. High GRK-2 levels are associated with hypertension. GRK-2 has a role in insulin secretion. GRK has a role in CNS signaling. GRK has a role in hormone secretion. GRK has a role in olfaction.

Any modulator of GAST will thus serve to change the level of GRK-associated signal transduction and thus will act modulate a metabolic parameters.

### Small Molecule Inhibitors

Low molecular weight organic molecules can act as inhibitors of GRK directly (by binding) to the kinase and by this inhibit the GAST. Such low molecular weight organic molecules are known in the art. Preferred low molecular weight organic molecules are GRK2 inhibitor H8, tri-fuorperazine, polyanions such as heparine and dextran sulfates.

### Ribozymes that specifically cleave GRK -RNA

A specific modulator of GAST is a ribozyme that is a catalytic oligonucleotide (typically RNA). The catalytic nucleotide can be tailored to specifically recognize, via hybridization, a specific mRNA region and thus cleave it and eliminate its expression. The ribozymes may be introduced to the cell as catalytic RNA molecules or as expression constructs for the expression of the catalytic RNA molecules.

### Antisense GAST Inhibitors

Another type of inhibitor of GAST is anti-sense nucleic acids. The nucleic acids are single stranded ribonucleic or deoxyribonucleic acid strands which contain nucleotides joined together through normal sugar-phosphate bonds. Antisense sequences can inhibit production of GRK protein by one of three mechanisms. By a first mechanism these antisense interfere with transcription as these antisense hybridize within the structural gene or in the regulatory region of the gene that encodes for GRK. This hybridization interrupts the transcription of GRK gene into mRNA. Since proper transcription or expression is effectively blocked by the hybridization of the anti-sense nucleic acids to the DNA, the kinase production is decreased and as a result of the depletion of the kinase the GAST is inhibited.

A second mechanism is the binding of the antisense in the cytoplasm to the mRNA, thus interfering with the formation of a proper translation construct leading to inhibition of translation of the mRNA to the protein. This leads to the decrease in the amount of GRK protein produced and thus to an inhibition of GAST.

A third mechanism is the formation of an mRNA-antisense duplex which leads to rapid degradation of mRNA duplex by RNases (such as Rnase. H). All these mechanisms lead to production of smaller amounts of GRK- produced by the cells than without the presence of these anti-sense nucleic acids, thus leading to GAST inhibition.

The particular nucleotides that are joined together to form the anti-sense sequence are those that are complementary to a region of the GRK structural gene, or complementary to regulatory region of the gene sufficient to inhibit production of functional GRK. These nucleotides of the anti-sense nucleic acids are specifically determined by the nucleotides of the target location and can easily be identified by the skilled practitioner once the sequence of the target location is established. The target location is a matter of choice to some extent. It lies within the region of the structural gene that encodes GRK or in the regulatory coding region of the structure. The target location nucleotide sequence can easily be established by the skilled practitioner from publicly available information concerning the GRK gene or can be obtained by routine examination of homologous genes coupled with standard molecular biology techniques.

By one option, the antisense is an oligonucleotide of several to several tens of nucleotides that are inserted into the cells. This is the preferred oligonucleotide in accordance with the invention. Typically the sequence is the first 20-25 nucleotides in the 5' terminal of the GRK cDNA (that are complementary to the mRNA). An example of such sequence is:
for GRK2 is: ctcggcctcg ggcgcggccg agcgccgcgc
and for GRK3 is: caagcttcat ctgtatttac agctgctcgc
or the RNA version of the above where T has been replaced by U.

Another option is the use of longer antisense sequences (up to several hundred nucleotides) by insertion into an expression vector, which can then transfected into cell by various gene transfer technologies. If that case the full sequence of the GRK can be used to construct a sequence which is complementary to it to produce a long antisense mRNA complementary to the native RNA. Finding the target of the kinase sequence to be used for antisense purposes may be carried out by screening through various overlapping sequences, or by use of various bio informative software that can locate likely targets in a given gene and give several alternative sequences for producing antisense sequences that can eliminate production.

### Negative Dominant Kinase Genes

Still another type of inhibitor of GAST is negative dominant GRK genes. The presence of these genes in cells allows non-functional GRK to be expressed to the exclusion of functional GRK. The negative dominant in the cells is inhibitory of GAST activity because this kinase is non-functional. Non-functional kinases, by definition, have no kinase activity. Negative dominant GRK genes are introduced into the cells by gene transfer techniques, which are becoming increasingly more standard in the art (calcium precipitation, electrical discharge, physical injection, use of carriers such as recombinant vectors, etc.). The introduced negative dominant GRK gene is incorporated in the cell's genome. There, copies of it are passed to progeny cells. Since this GRK- gene is negative dominant, it will be expressed in response to signals which induce GRK expression rather than the active form of GRK. Cells which have incorporated the negative dominant GRK gene will not be able to desensitize GPCR at the same levels as control because the expressed GRK is inactive. The negative dominant GRK genes can be found in the art or can be produced by standard gene mutation techniques which are well known to skilled practitioners in the art. These genes can be suitably packaged for transgenic procedures by appropriate methods and materials known to the skilled practitioners.

An example of a construct for dominant-negative GRK2 is expression vector pEF-GRK2-K220W (www.pharmci.org/scientificjournals/pharmaci/journal/2.htm1).

Another example of dominant-negative GRK are sequences coding for GRK wherein the codon for Lyn pr Lys which binds ATP in the catalytic unit, is replaced by codons coding for Ala or Met.

### Antibodies against GRK for Inhibitor GAST

A further type of inhibitors of GAST is antibodies that are immunoreactive with GRK. These antibodies bind to the kinase and thereby severely limit or prohibit its kinase activity or interrupt its interaction with other cellular components, all the above leading to GAST inhibition. The antibodies can be of any class or type. The binding site of the antibodies can be anywhere on the GRK molecule provided the immunoreactive binding between the antibody and the kinase molecule results in a severe inhibition of GAST. The antibodies can be polyclonal or monoclonal and are produced by techniques well-known to the skilled practitioner by using the GRK or immunogenic fragments thereof as the antigenic stimulus. The antibodies can be delivered to the individual by depositing suitable clonal cells which produce the antibodies, into the individual whose metabolism is to be modulated. These clonal cells secrete the antibodies into the bloodstream where they are carried to the target cancer cells for immunoreaction with the GRK proteins. Binding fragments of antibodies are also suitable provided they bind GRK with sufficient affinity so that the activity of the kinase is at least severely limited. Alternatively, the antibodies or suitable binding fragments can be introduced into the individual by any of a variety of techniques known to the skilled practitioner (physical injection, attachment to carriers that cross cell membranes, transgenic introduction into the prostate cancer cells for subsequent induction of expression, etc.). The secreted, introduced or expressed antibodies or suitable antibody fragments thereof immunoreactively bind to the GRK, thereby inhibiting their activity and thus GAST activity. Commercially available anti-GRK antibodies are available.

### Compounds comprising GRK Derived Peptides:

A further type of inhibitor of GAST is compounds comprising peptides, which herein are designated as *"GRK-derived peptides ".* These compounds comprising or consisting of said GRK-derived peptides are the preferred inhibitors of GAST in accordance with the invention, and thus are the preferred agents for the modulation of a metabolic parameter and for the treatment of metabolic associated conditions. The peptides apparently mimics a region in the kinase and thus bind to other cellular components with which the GRK interacts (such as the kinase substrates GPCRs). This binding interrupts the kinase-component interaction (especially kinase-substrate interaction) and thus inhibit GAST.

This GAST modulation leads to change in the desensitizing activity of GPCR by GRK -leading to modulation of metabolism.

The peptides according to the above non-limiting theory, mimic a region in the GRK kinase which is involved in the interaction of the GRK with other cellular components which are part of the GRK-associated signal transduction. Preferably, these cellular components are selected from: the substrates of GRK, other kinases, phosphatases, as well as co-factors and ATP. Thus, any peptide which mimics a part of the GRK responsible for said interaction can bind to the cellular component, and thus inhibit the GAST.

Specific preferred regions of the GRK that the GRK-derived peptides mimic are the HJ-loop, αD-loop, A-region, and B4-B5 region, as defined above, most preferably the region mimicked is the HJ-loop.

It is clear that for interruption of the kinase-cellular component interaction, there is no need to obtain a mimic of the full specific region of the kinase and a mimic of a subsequence that binds to the substrate in a competitor may be sufficient to interrupt said interaction, for example by steric hindrance. It is further clear that the interruption may be caused by mimicking of any one of several smaller subsequences present in the region so that there can be several alternative subsequences. It is further clear that for mimicking purposes it is not necessarily to obtain a sequence identical to the one present in the native kinase and variants of that sequence, that can faithfully copy the overall three dimensional structure of the region (when present in the full kinase) as well as the chemical characteristics of those side chains involved with interaction to the substrate, can also be used as mimics for interruption of the interaction. At times such variants may have better mimicking properties than the native sequence, as the variation may help stabilize the mimic amino acid sequence in a more favorable conformation, or may have stronger binding properties to the substrate.

The peptide derivative are short subsequences of at least five continuous amino acids obtained from the above sequences, as well as variants of the above sequences obtained by substitution of up to 40% of the amino acid with natural and non natural amino acids or with peptidomimetic moieties, and/or variants obtained by chemical modification of up to 40% of the amino acids, and/or deletions of up to 20% of the amino acids, provided that at least 50% of the amino acids are identical to the sequence of the parent protein, and provided that the variant maintains the biological properties of the parent sequence.

Most preferably, the sequence is at least five continuous amino acids obtained from the region of positions 382 to 414 HJ-loop, more preferably from positions 383 to 396 in said HJ-loop. The amino acid sequence may be a 5, 6, 7, 8, 9, 10, 11, 12, 13, 14, 15, 16, 17, 18, 19 and 20 amino acids. The sequence may be the sequence of a naturally appearing in the HJ-loop. However, actual empirical experiments show that sequences having substitutions at times have better GAST inhibiting properties than native sequences. Therefore, in the scope of the present invention are also included variants of the native sequence of the at least five continuous amino acids from the HJ-loop, in which up to 40% of the amino acids has been substituted, up to 40% of the amino acids have been chemically modified, and/or up to 20% have been deleted, provided that the variant shows at least 50% of its amino acid with the native sequence. In general, amino acids in the regions, and in particular the HJ-loop region, which are essential for GAST, should be either identical to those appearing in the native sequence, should be chemically modified or alternatively, should contain conservative substitutions (in the context of the present invention conservative substitutions also refer to substitutions by amino acids having the same steric properties, but when they replaced amino acid is charged, the substituted amino acid may be polar or hydrophobic as well). The other positions in the sequence may be replaced by conservative, non-conservative substitutions both by naturally and non naturally occurring amino acids as well as by organic peptidomimetics, these positions may be deleted or chemically modified.

In this invention, the peptides for inhibition of GAST are K024H001 (SEQ ID NO.: 1), K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.: 6); K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO.: 17), K024H901 (SEQ ID NO.: 18), and K024H903 (SEQ ID NO.: 19), as well as compounds comprising any one of SEQ ID NO:20 to SEQ ID NO:38.

### 1. Addition of non-peptidic groups to one or to both of the terminals of the GRK-derived peptides

Where the compound of the invention is a linear molecule, it is possible to place in any of its terminals various functional groups. The purpose of such a functional group may be for the improvement of the GAST inhibition. The functional groups may also serve for the purpose of improving physiological properties of the compound not related directly to GAST inhibition such as: improvement in stability, penetration (through cellular membranes or barriers), tissue localization, efficacy, decreased clearance, decreased toxicity, improved selectivity, improved resistance to repletion by cellular pumps, and the like. The functional groups may be also detectable labels added for diagnostic or research purposes. For convenience sake the free N-terminal of one of the sequences contained in the compounds of the invention will be termed as the N-terminal of the compound, and the free C-terminal of the sequence will be considered as the C-terminal of the compound (these terms being used for convenience sake). Either the C-terminus or the N-terminus of the sequences, or both, can be linked to a carboxylic acid functional groups or an amine functional group, respectively.

Suitable functional groups are described in Green and Wuts, *"Protecting Groups in Organic Synthesis",* John Wiley and Sons, Chapters 5 and 7, 1991, the teachings of which are incorporated herein by reference. Preferred protecting groups are those that facilitate transport of the compound attached thereto into a cell, for example, by reducing the hydrophilicity and increasing the lipophilicity of the compounds, these being a specific preferred example for *"a moiety for transport across cellular membranes ".*

These moieties can be cleaved *in vivo,* either by hydrolysis or enzymatically, inside the cell. (Ditter *et al., J. Pharm. Sci.* **57**:783 (1968); Ditter et al., J. Pharm. Sci. 57:828 (1968); Ditter et al., J. Pharm. Sci. 58:557 (1969); *King et al*., *Biochemistry* **26**:2294 (1987); Lindberg *et al., Drug Metabolism and Disposition* **17**:311 (1989); and *Tunek et al., Biochem. Pharm.* **37**:3867 (1988), Anderson *et al., Arch. Biochem. Biophys.* **239**:538 (1985) and Singhal *et al., FASEB J.* **1**:220 (1987)). Hydroxyl protecting groups include esters, carbonates and carbamate protecting groups. Amine protecting groups include alkoxy and aryloxy carbonyl groups, as described above for N-terminal protecting groups. Carboxylic acid protecting groups include aliphatic, benzylic and aryl esters, as described above for C-terminal protecting groups. In one embodiment, the carboxylic acid group in the side chain of one or more glutamic acid or aspartic acid residue in a compound of the present invention is protected, preferably with a methyl, ethyl, benzyl or substituted benzyl ester, more preferably as a benzyl ester.

In addition, a modified lysine residue can be added to the C-terminal of the compound to enhance biological activity. Examples of lysine modification include the addition of an aromatic substitute, such as benzoyl benzoic acid, dansyl-lysine various derivatives of benzoic acids (difluoro-, trifluromethy-, acetamido-, dimethyl-, dimethylamino-, methoxy-) or various derivatives of carboxylic acid (pyrazine-, thiophene-, pyridine-, indole-, naphthalene-, biphenyl,), or an aliphatic group, such as acyl, or a myristic or stearic acid, at the epsilon amino group of the lysine residue.

Examples of N-terminal protecting groups include acyl groups (-CO-R1) and alkoxy carbonyl or aryloxy carbonyl groups (-CO-0-R1), wherein R1 is an aliphatic, substituted aliphatic, benzyl, substituted benzyl, aromatic or a substituted aromatic group. Specific examples of acyl groups include acetyl, (ethyl)-CO-, n-propyl-CO-, iso-propyl-CO-, n-butyl-CO-, sec-butyl-CO-, t-butyl-CO-, hexyl, lauroyl, palmitoyl, myristoyl, stearyl, oleoyl phenyl-CO-, substituted phenyl-CO-, benzyl-CO- and (substituted benzyl)-CO-. Examples of alkoxy carbonyl and aryloxy carbonyl groups include CH3-O-CO-, (ethyl)-O-CO-, n-propyl-O-CO-, iso-propyl-O-CO-, n-butyl-O-CO-, sec-butyl-O-CO-, t-butyl-O-CO-, phenyl-O-CO-, substituted phenyl-O-CO- and benzyl-O-CO-, (substituted benzyl)- O-CO-. Adamantan, naphtalen, myristoleyl, tuluen, biphenyl, cinnamoyl, nitrobenzoy, toluoyl, furoyl, benzoyl, cyclohexane, norbomane, Z-caproic. In order to facilitate the N-acylation, one to four glycine residues can be present in the N-terminus of the molecule.

The carboxyl group at the C-terminus of the compound can be protected, for example, by an amide (i.e., the hydroxyl group at the C-terminus is replaced with -NH₂, -NHR₂ and -NR₂R₃) or ester (i.e. the hydroxyl group at the C-terminus is replaced with -OR₂). R₂ and R₃ are independently an aliphatic, substituted aliphatic, benzyl, substituted benzyl, aryl or a substituted aryl group. In addition, taken together with the nitrogen atom, R₂ and R₃ can form a C4 to C8 heterocyclic ring with from about 0-2 additional heteroatoms such as nitrogen, oxygen or sulfur. Examples of suitable heterocyclic rings include piperidinyl, pyrrolidinyl, morpholino, thiomorpholino or piperazinyl. Examples of C-terminal protecting groups include -NH₂, -NHCH₃, -N(CH₃)₂, -NH(ethyl), -N(ethyl)₂ , -N(methyl) (ethyl), -NH(benzyl), -N(C1-C4 alkyl)(benzyl), -NH(phenyl), -N(C1-C4 alkyl) (phenyl), -OCH₃, -O-(ethyl), -O-(n-propyl), -O-(n-butyl), -O-(iso-propyl), -O-(sec-butyl), -O-(t-butyl), -O-benzyl and -O-phenyl.

Preferably the compounds includes in the N-terminal a hydrocarbon having a length of C₄-C₂₀ preferably C₆-C₁₈, most preferably C₈-C₁₆. Example of hydrophobic moieties are: aaystyl, stearyl, lauroyl, palmitoyl and acetyl etc.

### 2. Finding a shorter subsequences of GRK-derived peptides

As indicated, GRK-derived peptides included in the compounds for inhibition of GAST, are obtained by finding which sequences from the above regions (HJ-loop, A-region, αD-region, B4-B5 region) that inhibit GAST. Typically it is desired ,for ease of synthesis and administration, to find the shortest sequence possible which is still active. In the following, the finding of the shortest sequence will be disclosed in connection with HJ-loop, but this description is applicable also to the other regions.

A shorter subsequence of the HJ-loop comprising a continuous stretch of at least five amino acid can be found by preparing a series of partially overlapping peptides each of 5-10 amino acids and each obtained by synthesizing a sequence that is one position removed from the previous sequence.

For example, the HJ-loop is in position 382-414, and it is to be desired to prepare 10 aa peptides, then the following, partially overlapping peptides are prepared, a peptide having the sequence 382-391, 383-392, 384-393, ... ... 405-414. The GAST inhibiting activities of the subsequences is then determined in a test assay. The best 10-aa peptide is then chosen.

For checking whether the 10 aa peptide can be reduced in sequence, it is possible to either repeat the above procedure (preparing a series of partially overlapping peptides) using 5 aa long peptides that span the length of the chosen 10 aa peptide, or to shorten the 10 aa peptide by deleting alternatively from each terminal, an amino acid, and testing the GAST inhibiting activity of the progressively truncated peptides, until the optimal sequence of at least 5, at least 6, at least 7, at least 8, at least 9 aa peptide is obtained or until it is determined that longer sequences are required. As the HJ-loop (as well as the other regions) is relatively small, typically the number of different peptides to be tested is also small. For example, for an HJ-loop having a length of about 20 aa, there is a need to prepare only 12 peptides to find the optimal 8 aa peptide. After the best 8-aa peptide is obtained, it is possible to delete sequentially amino acids from one or both terminals of the 8 per peptide for obtaining the shortest sequence of 5, 6 or 7 aa that is still active. For these steps only 16 sequences have to be tested, so that by testing only 24 peptides it is possible to find such a shorter sequence.

### 3. Identifying essential and non-essential amino acids in the subsequence chosen

### A. Ala-Scan

Once the shorter continuous stretch of at least 5 (at least 6, 7, 8, 9, 10, 11, 12 or 13) amino acids has been identified, as explained above, it is necessary to realize which of the amino acids in the stretch are essential (i.e. crucial for the kinase-associated signal transduction modulation) and which are non-essential. Without wishing to be bound by theory, in almost every native protein involved in interaction with other cellular components, some amino acids are involved with the interaction (essential amino acids) and some amino acids are not involved in the interaction (non-essential amino acids), for example since they are cryptic and inaccessible. A short peptide which is to mimic a region of the GRK protein behaves in the same way as the region when present in the full kinase: some amino acids actually interact with the substrate (or other interacting components) and other amino acids merely serve to spatially position the interacting amino acids, but do not participate in the interaction with the other cellular components.

Essential amino acids have to be maintained (i.e. be identical to those appearing in the native kinase), or replaced by conservative substitutions (see definition below) to obtain variants of the peptides or can be chemically modified. Non-essential amino acids can be maintained, deleted, chemically modified, replaced by a spacer or replaced by conservative or non-conservative substitutions.

Identification of essential vs. non-essential amino acids in the peptide can be achieved by preparing several peptides that have a shorter sequence than the full region (see 2 above) in which each amino acid is sequentially replaced by the amino acid Ala (*"Ala-Scan."),* or sequentially each amino acid is omitted *("omission-scan").* This allows to identify the amino acids which modulating activity is decreased by said replacement/omission *("essential")* and which are not decreased by said replacement/omission("*non-essential"*) (Morrison *et al., Chemical Biology* **5**:302-307,2001). Another option for testing the importance of various peptides is by the use of site-directed mutagenesis. Other Structure-Activity-Relationship (SAR) techniques may also be used.

### B. 3-D analysis

Another strategy for finding essential vs. non-essential amino acids is by determining which aa of the A-region, in the 3D of the full kinase are exposed and which are cryptic.

Typically cryptic aa are non-essential and exposed or partially exposed are more likely to be essential. However, if one wishing to "*guess*" theoretically which *"non-conservative"* substitutions in the cryptic region can be tolerated, a good guideline is to *"check"* on a 3D computer model of the full kinase, whether these changes drastically alter the overall shape of the regions when the "altered" peptide is superimposed on the full kinase.

### 5. The use of the claimed compounds for the preparation of medicines

The inhibitor of GAST of the present invention, or the compounds comprising the GRK-derived peptides defined in the claims can be used as active ingredients (together with a pharmaceutically acceptable carrier) to produce a pharmaceutical composition. The pharmaceutical composition may comprise one, or a mixture of two or more of the different. GAST inhibitors of the invention in an acceptable carrier.

The pharmaceutical composition should be used for the treatment of any disease, disorder, or condition, wherein a beneficial therapeutical effect may be evident by the modulation of at least one metabolic parameter, especially for the treatment of a disease selected from :diabetes, obesity, dislipidemia, hypertension, syndrome-X associated phenomena.

The GAST modulators of the present invention can be administered parenterally. Parenteral administration can include, for example, systemic administration, such as by intramuscular, intravenous, subcutaneous, or intraperitoneal injection. Compounds which resist proteolysis can be administered orally, for example, in capsules, suspensions or tablets. The compound can also be administered by inhalation or insufflations or via a nasal spray.

The GAST inhibitors can be administered to the individual in conjunction with an acceptable pharmaceutical carrier as part of a pharmaceutical composition for treating the diseases discussed above. Suitable pharmaceutical carriers may contain inert ingredients which do not interact with the compounds. Standard pharmaceutical formulation techniques may be employed such as those described in Remington's Pharmaceutical Sciences, Mack Publishing Company, Baston, PA. Suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like. Methods for encapsulating compositions (such as in a coating of hard gelatin or cyclodextran) are known in the art (Baker, *et al., Controlled Release of Biological Active Agents,* John Wiley and Sons, 1986). The formation may be also resources for administration to skin, or in the form of salve, solution, ointment, etc. for topical administration.

The composition may be administered locally to the site of activity such as administered directly into the heart.

The pharmaceutical compositions may also be administered in conjunction with other modes of metabolic therapy, for example, with other compounds used to treat metabolic diseases, such as insulin, as it may work synergistically with insulin and this decrease the amount of insulin required, or decrease the frequency of insulin administration.

A *"therapeutically effective amount"* is the quantity of compound which results in an improved clinical outcome as a result of the treatment compared with a typical clinical outcome in the absence of the treatment. *An "improved clinical outcome"* results in the individual with the disease experiencing fewer symptoms or complications of the disease, including a longer life expectancy, as a result of the treatment. With respect to diabetes, an *"improved clinical outcome"* refers to decreased blood glucose levels, improved responses to insulin, starvation, stress, or glucose load in the presence of the compound of the invention, a longer life expectancy, a reduction in the complications of the disease (e.g., neuropathy, retinopathy, nephropathy and degeneration of blood vessels associated with diabetes) and an improved quality of life, as described above. Another aspect of an improved clinical outcome is a reduction in medication dosage (e.g., a reduction in insulin or other hypoglycemic agent needed to maintain adequate blood glucose levels).

With respect to obesity, an improved clinical outcome refers to increased weight reduction per calorie intake. It also refers to a decrease in the complications which are a consequence of obesity, for example heart disease such as arteriosclerosis and high blood pressure. With respect to syndrome X an improved clinical outcome refers to a longer life expectancy, a reduction in the incidence or severity of the different mobilities included in the syndrome (e.g., ischemic heart disease, atherosclerosis, type II DM and obesity) and an improved quality of life. With respect to other manifestations of syndrome X, it includes lowering of blood pressure and improvement of serum lipid and cholesterol profile.

### 6. Determination of GAST modulating activity

It should be appreciated that some of the compounds that comprise sequences (a) - (i) above are better GAST modulators than others. Some of the conservative substitutions in the essential positions may diminish the modulating, while other such conservative substitution in the essential positions may improve these modulating activities. The same is true also for deletions, substitutions (both conservative and non-conservative) in non-essential positions, as well as to chemical modifications (in any position) or insertions. In addition the type and size of the non-amino acid portion of the compounds, such as a hydrophobic moiety in one of its terminals may diminish or increase the GAST modulating activities. The GAST modulating activities that can be determined for example by using one of the assays stipulated below.

### 6.1 Cellular Assays

It can be readily determined whether a compound modulates the activity of a GAST by incubating the compound with cells which have one or more cellular activities controlled by the GAST. Examples of these cellular activities include cell proliferation, cell differentiation, cell morphology, cell survival or apoptosis, cell response to external stimuli, gene expression, lipid metabolism, glycogen or glucose metabolism and mitosis, secretion or production of compounds by the cells. The cells are incubated with the candidate compound to produce a test mixture under conditions suitable for assessing the level of the GAST. The activity of the GAST is assessed and compared with a suitable control, e.g., the activity of the same cells incubated under the same conditions in the absence of the candidate compound (or in the presence of a control compound). A lesser activity of GAST in the test mixture compared with the control indicates that the candidate compound modulates GAST.

Suitable cells for the assay include normal cells which express the GRK-, cells which have been genetically engineered to express a GRK, malignant cells expressing a GRK or immortalized cells that express the kinase.

Conditions suitable for assessing activity include conditions suitable for assessing a cellular activity or function under control of the GAST pathway. Generally, a cellular activity or function can be assessed when the cells are exposed to conditions suitable for cell growth, including a suitable temperature (for example, between about 30°C to about 42°C) and the presence of the suitable concentrations of nutrients in the medium (e.g., amino acids, vitamins, growth factors or of specific activators such as cytokines, hormones and the like).

For example, the activity may be assessed by measuring melanogenesis by melanocytes as in Example 3 below. Another cellular assay is determination of cAMP in C6 glioblastoma cells as indicatedin example 4 bellow..

### 6.2 Phosphorylation of substances

It is possible to assess the GAST activity and the changes in this GAST as compared to control, by determining the phosphorylation level of the substrate proteins of the GRK. Examples of possible GRK substrates are: tubuline, β-adrenergic receptor, α₂adrenergic receptor, acetylcholine receptor, d-opioid-receptor and µ-opioid-receptor as β_{2/1}-adrenergic receptor, α₂-adrenergic receptor, acetylcholine receptor, d-opioid receptor, rhodopsin ,A_{(1,2.3)}-purinergic receptor,, synuclein, Angiotensin II 1a, DA-dopamine, N-formyl peptide, muscarinic receptor, platelet activating factor, thrombin (Bunemann *et al, J. of Physiology* (1999) 517.1,5-23) Cells known to express the GRK such as for example are incubated with a candidate compound for inhibiting the GAST. Then the cells are lysed, the protein content of the cells is obtained and separated on a gel. The substrates can be identified by use of suitable molecular weight markers, or by using suitable antibodies, reactive against the specific GPCR used. The level of phosphorylation of the substrate may be determined by suing labeled anti-Tyr antibodies. Alternatively, the suitable substrate may be immuno-precipitated using antibodies. The level of substrate phosphorylation in the immuno- precipitate can be determined by using anti-phosphotyrosine antibodies (see Fujimoto *et al., Immunity,* **13**:47-57 (2000)).

By another option, phosphorylation may be determined in a cell-free system by incubating a mixture comprising GRK, the substrate of the kinase (the suitable GPCR) and candidate molecules for inhibiting GAST in the presence of ATP under conditions enabling phosphorylation. The proteins are then subjected to gel separation, transferred to nitrocellulose where the substrate band is identified by antibody or molecular weight marker followed by immunoblotting by anti-phosphotyrosine antibody. Alternatively it is possible to use [γ-³² P] ATP and quantify the amount of radioactivity incorporated in the substrate (See Fujimoto *et al., The J. of Immunol.* 7088-7094 (1999).

### 6.3. Tissue or in vivo Assay

Suitable assays for determining inhibition of GAST can be by inducing diabetes in animals for example, by injection of streptozotoun that destroys some of the insulin secreting cells (as long as some insulin is maintained) or by using animal models which have a tendency to develop diabetes, obesity, high serum lipid profile, hypertension, either naturally (sand rat, ob/ob mice etc.) or through genetic engineering, and then determining the change of glucose level in the blood of the animal. Other examples are determination of change of weight of appetite in the treated animals.

### 7. Preparation of Antibodies

The GRK-derived peptides of the present invention can be useful in the preparation of specific antibodies against GRK. Suitable antibodies can be raised against a GRK peptide by conjugating the peptide to a suitable carrier, such as keyhole limpet hemocyanin or serum albumin; polyclonal and monoclonal antibody production can be performed using any suitable technique. A variety of methods have been described (see e.g., Kohler *et al., Nature,* **256:**495-497 (1975) and *Eur. J. Immunol.* **6**:511-519 (1976); Milstein et al., Nature 266: 550-552 (1977); Koprowski *et al.,* U.S. Patent No. 4,172,124; Harlow, E. and D. Lane, 1988, *Antibodies:* A Laboratory Manual, (Cold Spring Harbor Laboratory: Cold Spring Harbor, NY); Current Protocols In Molecular Biology, Vol. 2 (Supplement 27, Summer 1994), Ausubel, F.M. *et al.,* Eds., (John Wiley & Sons: New York, NY), Chapter 11, (1991)). Generally, a hybridoma can be produced by fusing a suitable immortal cell line (e.g., a myeloma cell line such as SP2/0) with antibody producing cells. The antibody producing cell, preferably those of the spleen or lymph nodes, can be obtained from animals immunized with the antigen of interest. The fused cells (hybridomas) can be isolated using selective culture conditions, and cloned by limiting dilution. Cells which produce antibodies with the desired specificity can be selected by a suitable assay (e.g., ELISA).

The antibodies can be used to determine if an intracellular GRK is present in the cytoplasm of the cell. A lysate of the cell is generated (for example, by treating the cells with sodium hydroxide (0.2 N) and sodium dodecyl sulfate (1%) or with a non-ionic detergent like NP-40, centrifugating and separating the supernatant from the pellet), and treated with anti-GRK peptide antibody specific for GRK. The lysate is then analyzed, for example, by Western blotting or immunoprecipitation for complexes between GRK and antibody. Anti-GRK peptide antibodies can be utilized for the study of the intracellular distribution (compartmentalization) of GRK under various physiological conditions via the application of conventional immunocytochemistry such as immunofluorescence, immunoperoxidase technique and immunoelectron microscopy, in conjunction with the specific anti-GRK peptide antibody.

Antibodies reactive with the GRK peptides are also useful to detect and/or quantitate the GRK in a sample, or to purify the GRK (e.g., by immunoaffinity purification).

The GRK-derived peptides of the present invention can also be used to identify ligands which interact with GRK and which inhibit the activity of GRK. For example, an affinity column can be prepared to which a GRK peptide is covalently attached, directly or via a linker. This column, in turn, can be utilized for the isolation and identification of specific ligands which bind the GRK peptide and which will also likely bind the GRK. The ligand can then be eluted from the column, characterized and tested for its ability to inhibit GRK function.

### 8. Preparation of the Compounds

Peptide sequences for producing any of the sequence of the compounds of the invention may be synthesized by solid phase peptide synthesis (e.g., t-BOC or F-MOC) method, by solution phase synthesis, or by other suitable techniques including combinations of the foregoing methods. The t-BOC and F-MOC methods, which are established and widely used, are described in Aarifield, *J. Am. Chem. Soc.,* **88**:2149 (1963); Meienhofer, Hormonal Proteins and Peptides, C.H. Li, Ed., Academic Press, 1983, pp. 48-267; and Barany and Aarifield, in The Peptides, E. Gross and J. Meienhofer, Eds., Academic Press, New York, 1980, pp. 3-285. Methods of solid phase peptide synthesis are described in Aarifield, R.B., *Science,* **232**:341 (1986); Carpino, L.A. and Han, G.Y, *J*. *Org. Chem.,* **37**:3404 (1972); and Gauspohl, *H. et al., Synthesis,* **5**:315 (1992)). The teachings of these references are incorporated herein by reference.

As indicated above the compounds of the invention may be prepared utilizing various peptidic cyclizing techniques. Methods of cyclizing compounds having peptide sequences are described, for example, in Lobl *et al.,* WO 92/00995, the teachings of which are incorporated herein by reference. Cyclized molecules can be prepared by protecting the side chains of the two amino acids to be used in the ring closure with groups that can be selectively removed while all other side-chain protecting groups remain intact. Selective deprotection is best achieved by using orthogonal side-chain protecting groups such as allyl (OAI) (for the carboxyl group in the side chain of glutamic acid or aspartic acid, for example), allyloxy carbonyl (Aloc) (for the amino nitrogen in the side chain of lysine or ornithine, for example) or acetamidomethyl (Acm) (for the sulfhydryl of cysteine) protecting groups. OAI and Aloc are easily removed by Pd and Acm is easily removed by iodine treatment.

Other modes of cyclization (beyond N- to C- terminal cyclization) may include: N- to backbone cyclization, C- to backbone cyclization, N- to side chain cyclization, C- to side chain cyclization, backbone to side chain cyclization, backbone to backbone cyclization and side chain to side chain cyclization.

### Example 1 - Preparation of Compounds comprising GRK-derived peptides

The compounds of this invention can be synthesized utilizing a 430A Peptide Synthesizer from Applied Biosystems using F-Moc technology according to manufacturer's protocols. Other suitable methodologies for preparing peptides are known to person skilled in the art. See e.g., Merrifield, R.B., *Science, 232:* 341 (1986); Carpino, L.A., Han, G.Y., *J. Org. Chem., 37*: 3404 (1972); Gauspohl, H., et *al., Synthesis,* 5: 315 (1992)). The teachings of which are incorporated herein by reference.

Rink Amide Resin [4(2',4' Dimethoxyphenyl-FMOC amino methyl) phenoxy resin] was used for the synthesis of C-amidated peptides. The alpha-amino group of the amino acid was protected by an FMOC group, which was removed at the beginning of each cycle by a weak base, 20% piperidine in N-methylpyrrolidone (NMP). After deprotection, the resin was washed with NMP to remove the piperidine. *In situ* activation of the amino acid derivative was performed by the FASTMOC Chemistry using HBTU (2(1-benzotriazolyl-1-yl)-1,1,3,3-tetramethyluronium) dissolved in HOBt (1-hydroxybenzotriazole) and DMF (dimethylformamide). The amino acid was dissolved in this solution with additional NMP. DIEA (diisopropylethylamine) was added to initiate activation. Alternatively, the activation method of DCC (dicycbohexylcarbodiimide) and HOBL was utilized to form an HOBt active ester. Coupling was performed in NMP Following acetylation of the N-terminus (optional), TFA (trifluoroacetic acid) cleavage procedure of the peptide from the resin and the side chain protecting groups was applied using 0.75 g crystalline phenol; 0.25 ml EDT (1,2-ethandithiol); 0.5 ml thioanisoie; 0.5 ml D.I. H₂O; 10 ml TFA.

### Example 2 Type II Diabetes in Sand Rats (psamomys)

Sand-rats (psamomys) which are genetically prone to develop Type II diabetes were used in this study. The genetically selected sand-rats, 3 to 6 months old, were fed an energy-rich diet (Weizmann HE) for about 3 to 10 days until they became diabetic, as judged by their elevated blood-glucose level (see R. Kalman *et al.,* "The Efficiency of Sand Rat Metabolism is Responsible for Development of Obesity and Diabetes", *J*. *of Basic & Clinical Physiology & Pharmacology* (1993), vol. 4, no. 1-2, pp 57-68, the pertinent portions of which are incorporated herein by reference).

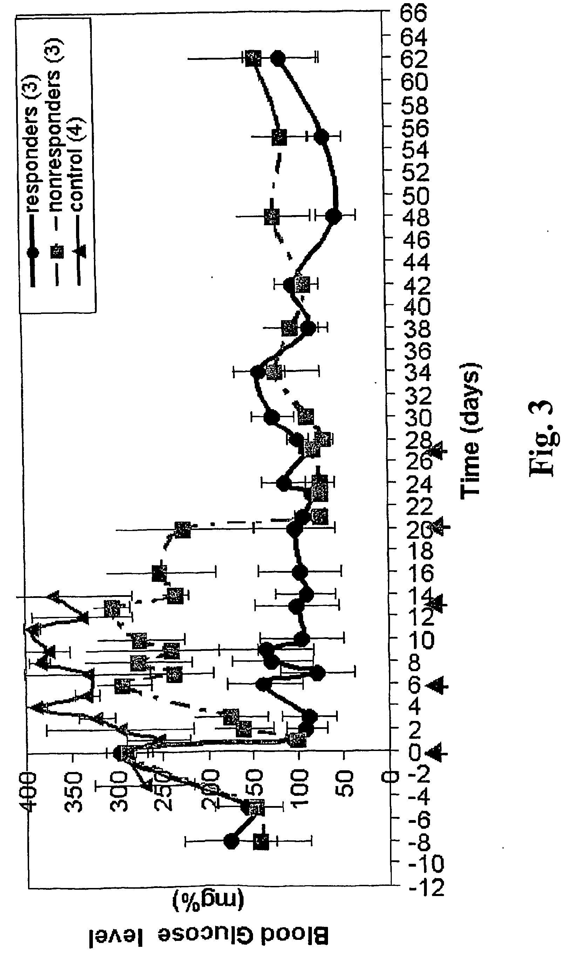

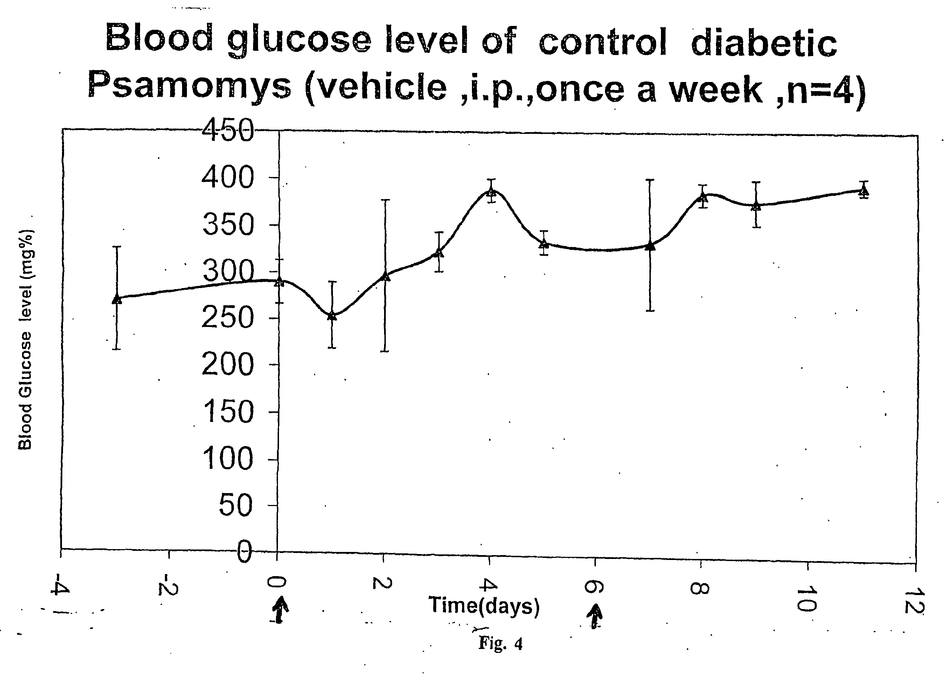

The diabetic sand-rats were injected i.p. once a week with a compound comprising GRK-derived peptide K024H107 (SEQ ID NO:10) at a dose of 10 mg/kg. The peptide was prepared by diluting a 10 mM solution of the peptide in 100% DMSO with phosphate buffered saline (PBS) containing 0.1% bovine serum albumin (BSA) to a concentration of 400 µM. Forty µM of the 10 mM peptide in DMSO solution was mixed with 160 µl of 1.6M NH₄HCO₃ and heated for 40 minutes at 100°C. The resultant solution was then diluted to 400 µM in 2 M Hepes buffer (pH 7.0). This peptide stock solution was labeled *"tbi ".* The vehicle of the solution for injection included 8% DMSO, 0.67M ammonium bicarbonate, and 2M Hepes. Control animals received an i.p. injection of the vehicle only. The results for treated animals are shown in fig 3. and for control animals are shown in Fig. 4.

As can be seen from Figs. 3 and 4, after a single injection of the compound comprising the GRK-derived peptide there was dramatic decrease in blood-glucose to the normal level (Fig. 3), while no change was observed in the controls (Fig. 4). Additionally, in the treated group, it was noted that 4 animals became normoglycemic already after the first injection (responders, Fig. 3), and the rest of the treated animals also became normoglycemic after three additional weekly injections *( "nonresponders",* Fig. 3).

### Example 3 Measurement of Melanogenesis by Melanocytes in Cell Culture

Murine B16 melanoma cells were grown in DMEM + 10% FCS + 2mM Glutamine + 100units/ml Penicillin + 0.1mg/ml Streptomycin. The cells were incubated under controlled conditions (37°C, 5% CO₂).

The melanoma cells were plated in 96-well microtiter plates, 5,400 cells per well, and allowed to grow for 24 hours. Selected compounds comprising GRK-derived peptides were solubilized in DMSO and then diluted in PBS + 0.1% BSA to 10X of the final concentration (see the procedure in Example 2).

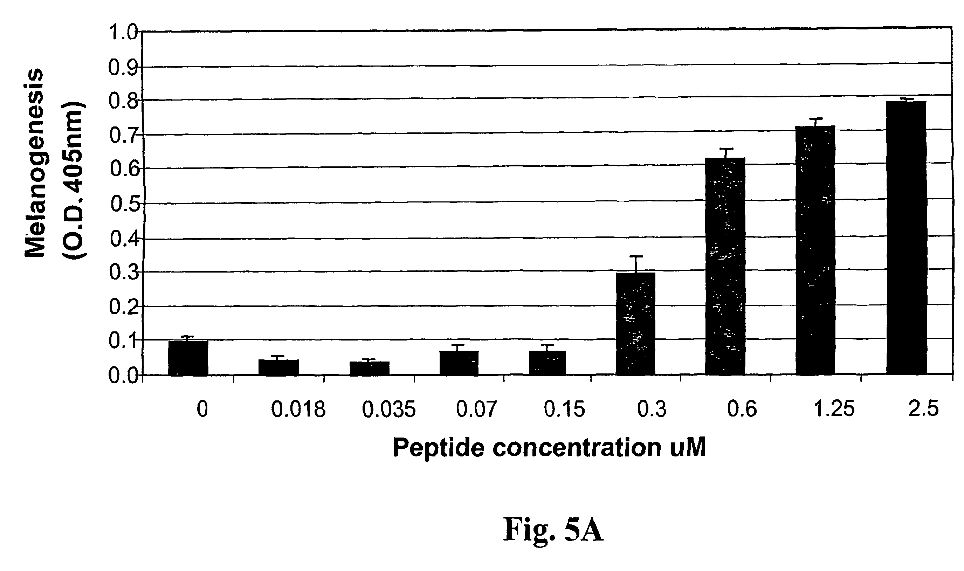

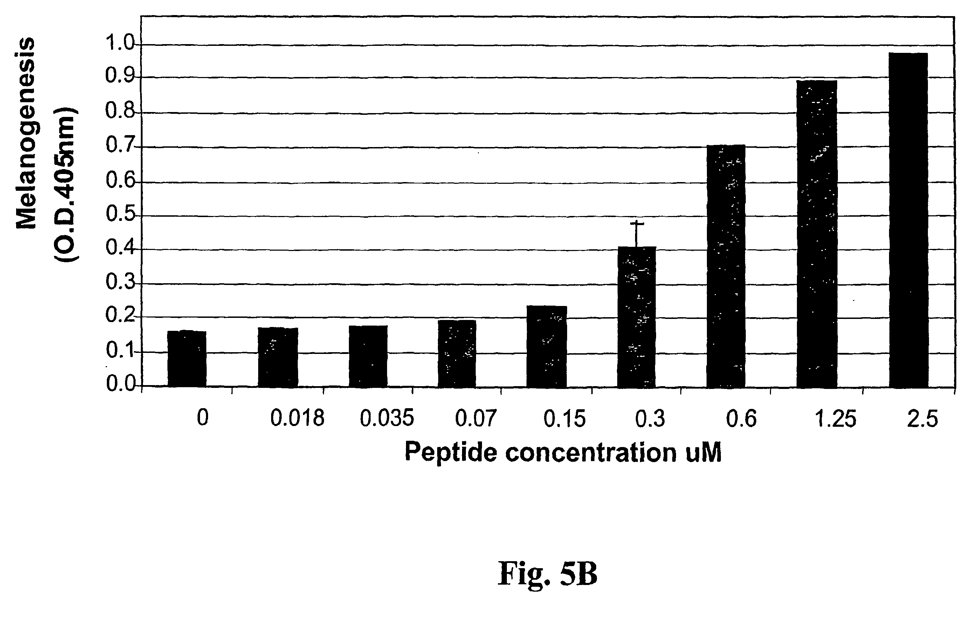

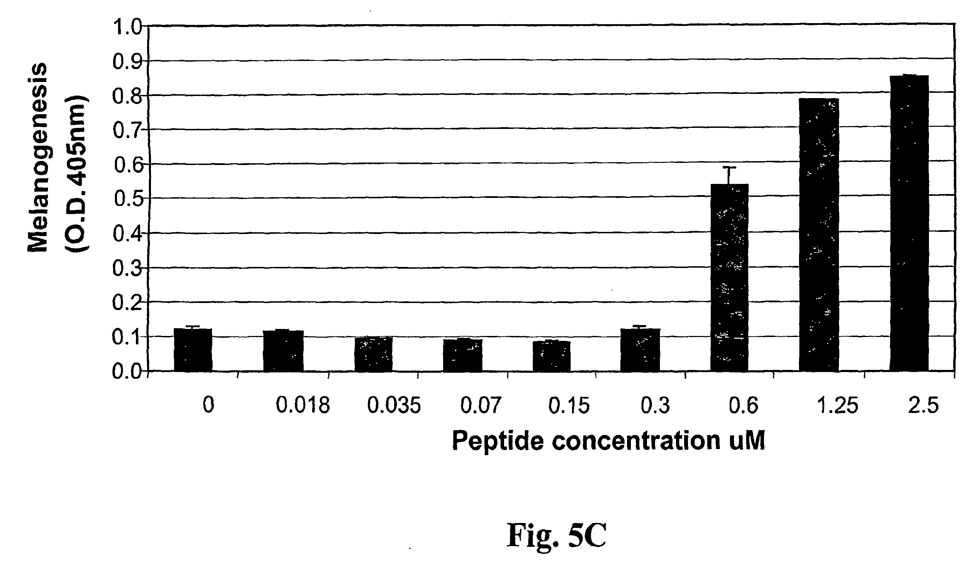

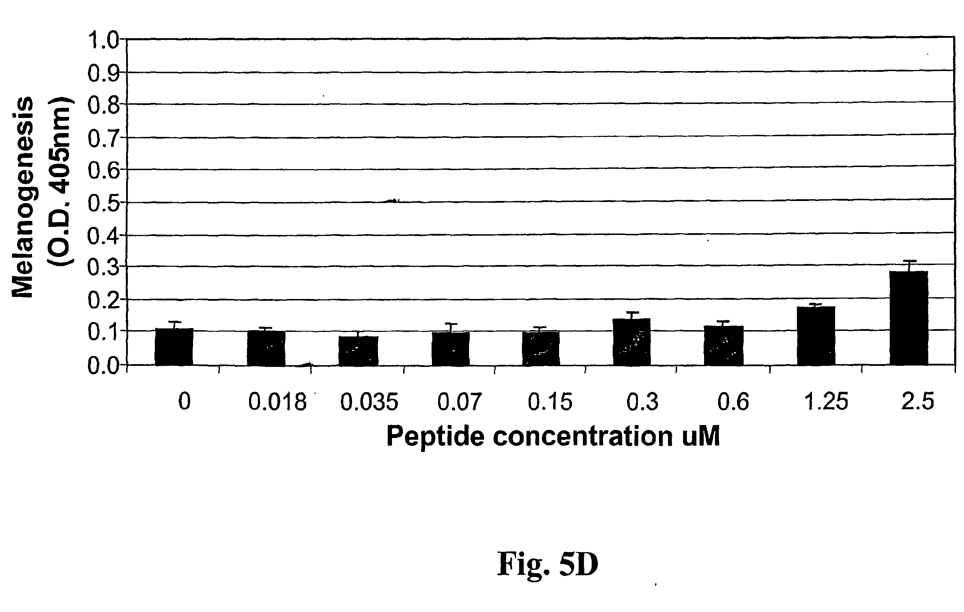

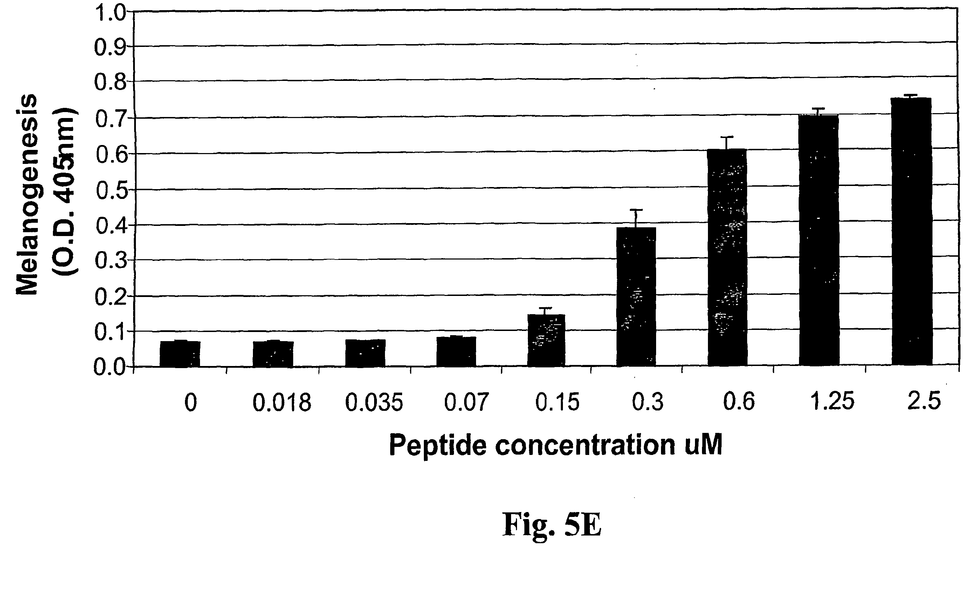

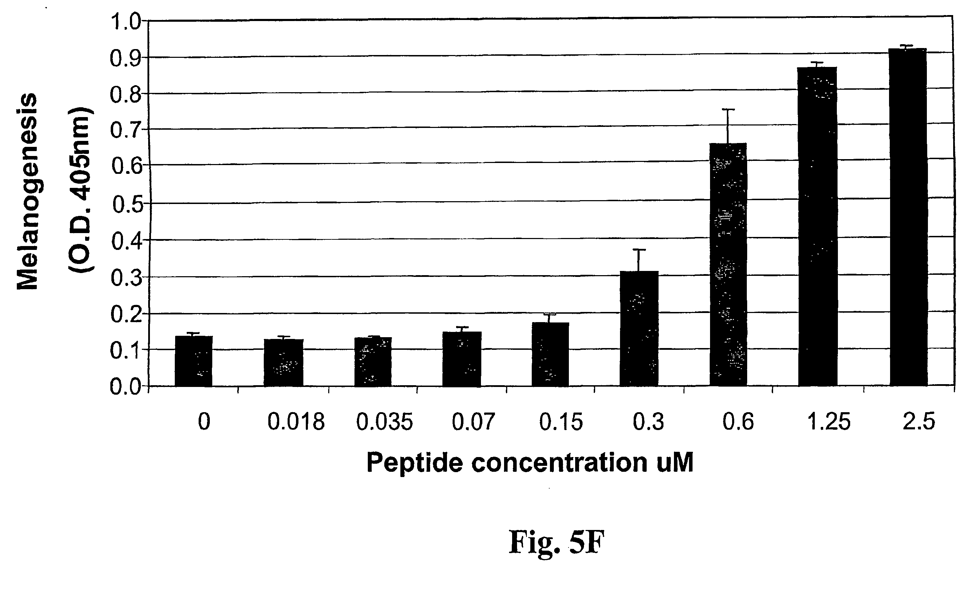

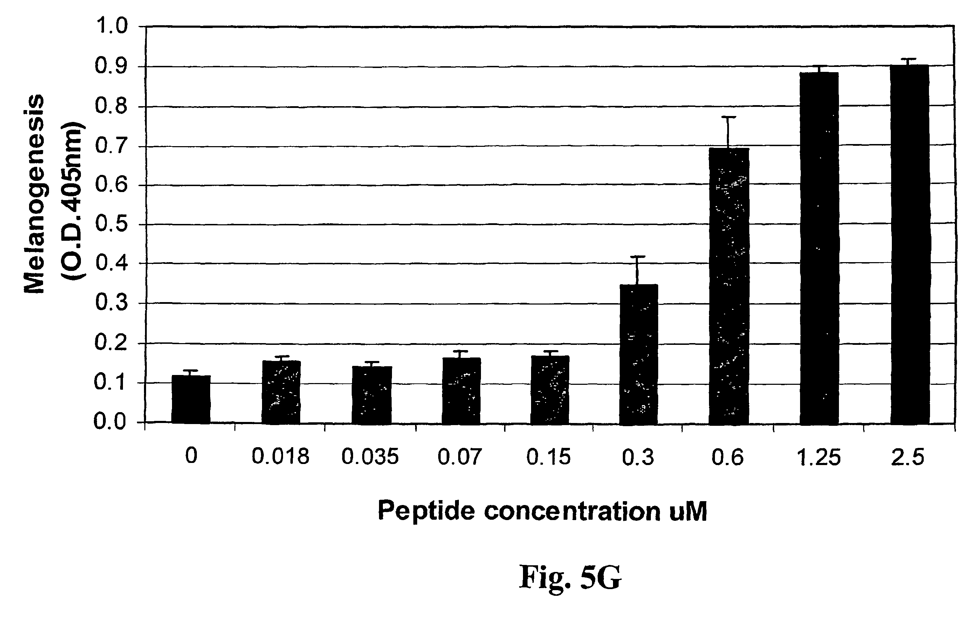

Six different compounds comprising different GRK-derived peptides were added to the corresponding wells at the stated final concentrations (see Figs. 5A-5F). The vehicle containing equal concentrations of DMSO, PBS and BSA was used as the control. The cells were then incubated for an additional 4-days, when dark melanin pigment accumulated in the wells of the treated cells.

Melanogenesis was then assessed by addition of 70 µl 1N NaOH per well to release all melanin from the cells and the optical density was determined by 405 nm, using an ELX-800 ELISA plate reader. Six wells were used for each concentration.

The results are shown in Figs. 5A-5G. It can be seen from these graphs that significant melanogenesis occurred at peptide concentrations of 0.6 µM and, for some peptides, as low as 0.15 µM. It is readily apparent from these graphs that these peptides cause an enhancement in melanogenesis from melanocytes and that this effect is evident by rhe use of several different GRK-derived peptides..

### Example 4 Inhibition of Cyclic-AMP production

It is well known that after activation of the various GPCRs there is an activation of the adenolate cyclase by G-proteins. This raises the cyclic-AMP levels in the cell, and these levels are then decreased due to the desensitization of the receptors by GRK. Thus it is expected that inhibition of GAST will eliminate this decrease in the cyclic AMP levels, and will cause a constitutive increase in cAMP levels.

C6 glioblastoma cells, which are known to express the β-adrenagic receptor (the substrate of GRK) have been used.

To the cells the following compounds were added:
- Group I:: Isoproteranol (ISO) (agonist of the β-adrenergic receptor) in concentrations of 1 µM and 10 µM;
- Group II:: The compound of the invention K024H107 (SEQ ID NO:10) in concentrations of 1µM and 10 µM;
- Group III:: A combination of the peptide KO24H107 at a concentration of 10µM and an hour later 1 µM isoproterenol;
- Group IV:: A first addition of 1µM isoproterenol and one hour later 10µM of the compound of the invention KO24H107

The amount of the activity was measured by measuring the increase of intracellular cyclic AMP levels, which were measured using the commercially available kit of Biotrack Cellular Communication Assay cyclic-AMP enzyme - immunoassay (EIA) system.

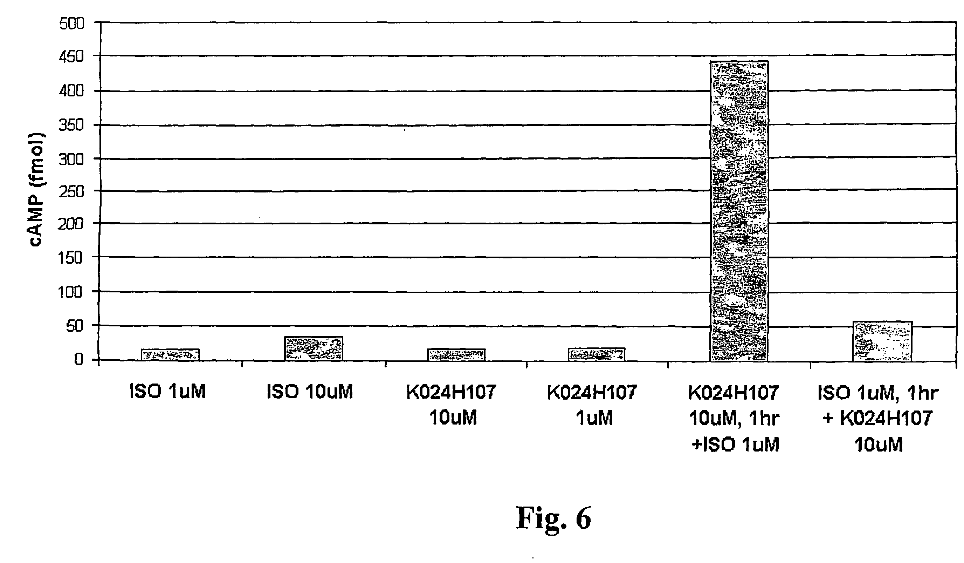

The results are shown in Fig. 6. As can be seen, the cyclic AMP levels rose significantly when initially the compound of the invention K024H107 was added, and an hour later isoproterenol (activator of the receptor) was added. When the order of addition was reversed, the result was significantly different, the cyclic AMP level hardly changed, probably due to the fact that there is a lag in the activity of the compound of the invention due to the time required for penetration through cellular membranes. This experiment clearly indicates that the compounds of the invention are capable of significantly increasing the signal transduced by GPCR.

### Example 5 Activity Of Compounds Of the Invention on Reduction of Weight And Appetites Of Normal Mice

18 Sabra male mice, 8 weeks old, were used and were divided into four groups as followed:
- Group I:: (n=5) served as control and were injected with a vehicle which were 1.1 % Tween80, 0.1 % BSA, in DDW.
- Group II:: (n=4) were injected with NDP-αMSH, a known regulator of weight, in a concentration of 0.9 µg/kg BW;
- Group III:: (n=5) were injected with the compound of the invention K024H107 at a concentration of 21 mg/kg body weight;
- Group IV:: (n=4) received a combination of NDP-αMSH and K024H107 at the same concentration as above.

The animals were weighed before trial and periodically during the trial.

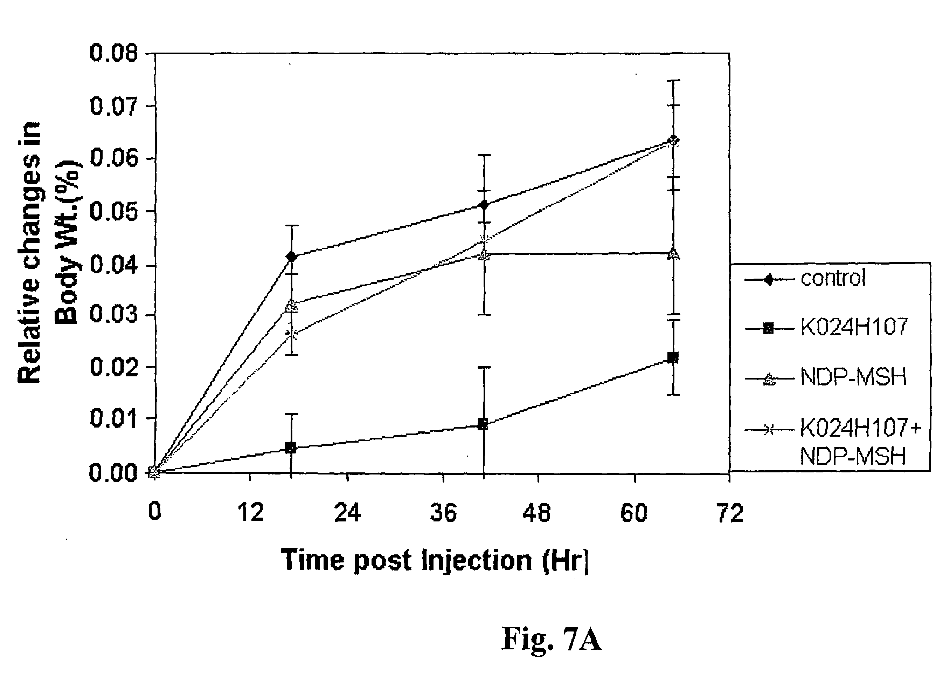

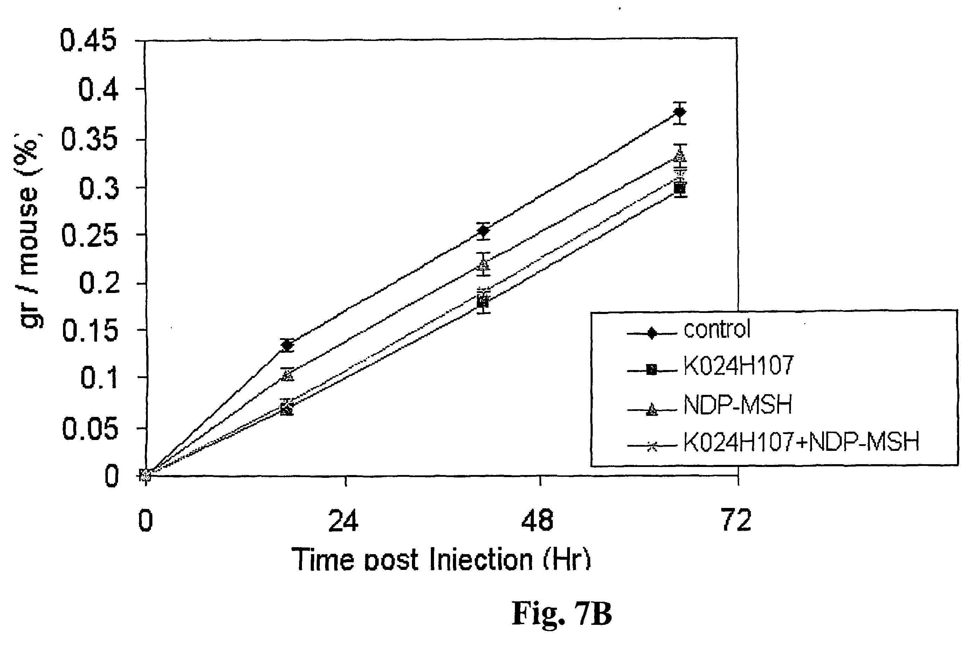

At the same time, food was weighted before it was introduced into cages and at the end of each tested period. From this point on, the follow-up included both weighing of the mice and of the food for the short period, every couple of hours, until night, and later on for the long period, each 24 hour cycle. The results are shown in Fig. 7A and 7B.

The results of Fig. 7A are shown as the percentage of change in body weight as compared to the initial weight for each mice, and then averaged for each group. The calculation of food consumption (Fig. 7B) was calculated in each group relative to the total weight of all animals (4 or 5 animals) and averaged separately for each group. As can be seen from the results, all three treated groups showed weight decrease and decrease in food consumption as compared to control. Group III treated with the compound of the invention alone showed the strongest effect.

The above experiment was conducted again with another compound comprising a GRK-derived peptide K024H112 (SEQ ID NO:5) 16 animals were used as follows:
- Group I:: (n=5) Vehicle as above;
- Group II:: (N=6) K024H112 23.2 mg/kg;
- Group III:: KO24H11231.5 mg/kg.

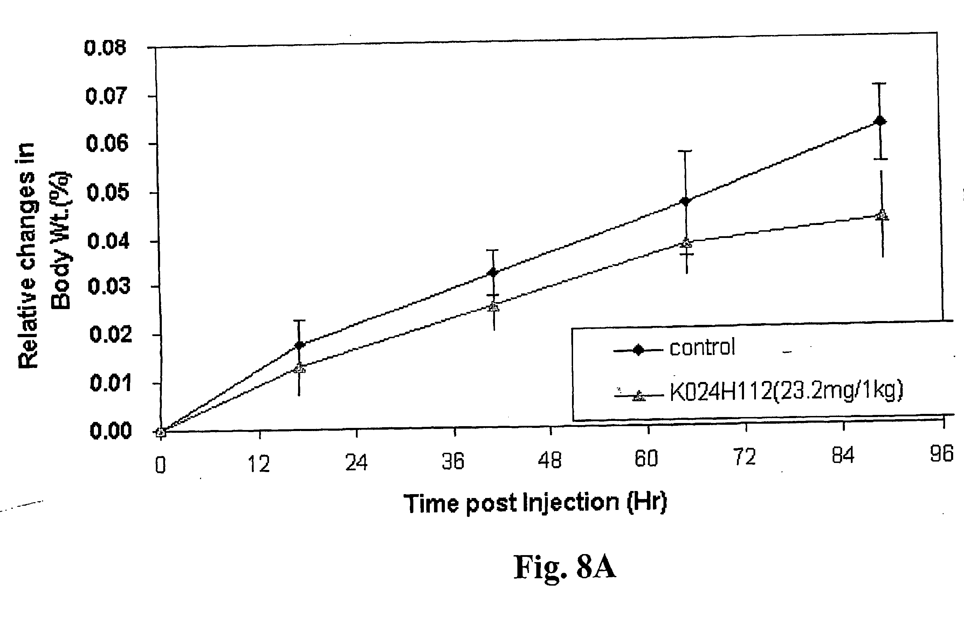

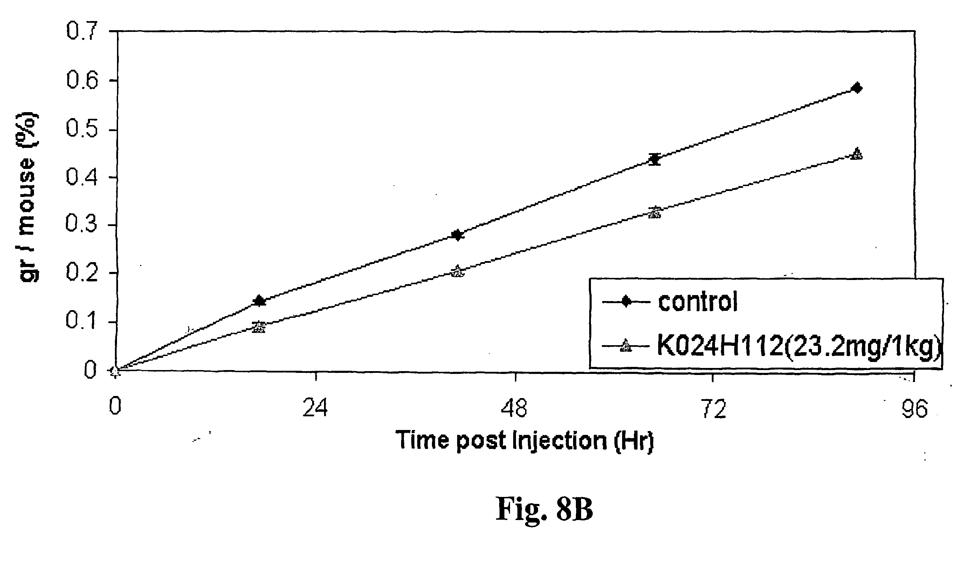

The results for weight change are shown in Fig 8A and of change in food consumption are shown in Fig. 8B. As can be seen, this compound was also effective in reducing both body weight and consumption.

The above results indicated that two different compounds of the inventions comprising two different GRK-derived sequences were also effective in reducing both body weight and consumption.

## Claims

1. A compound, **characterized in that** the compound is K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.: 6), K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO.: 17), K024H901 (SEQ ID NO.: 18), K024H903 (SEQ ID NO.: 19), or SEQ ID NO: 20 to SEQ ID NO: 38.

2. The compound according to claim 1, wherein the compound is K024H107 (SEQ ID NO.: 10).

3. Use of a compound according to claim 1 or claim 2 for the preparation of a medicine.

4. Use of a compound according to claim 3, wherein the medicine is for treating obesity, dyslipidemia, cholesterolemia, coagulation disorders, syndrome X, diabetes, hypertension or ateriosclerosis.

5. Use of at least one inhibitor of GRK-associated signal transduction (GAST) for the preparation of a medicament for the treatment of a diabetic-associated phenomena, wherein GAST inhibitor is K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.: 6), K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO.: 17), K024H901 (SEQ ID NO.: 18), K024H903 (SEQ ID NO.: 19), or SEQ ID NO.: 20 to SEQ ID NO.: 38.

6. The use according to claim 4, wherein the GAST-inhibitor is K024H107 (SEQ ID NO:10).

7. The use according to claim 4 or claim 5, wherein the diabetic associated phenomenom is diabetes type II, diabetic-associated obesity, diabetic related hypertension or diabetic associated dyslipidemia.

## Patentansprüche

1. Eine Verbindung, **dadurch gekennzeichnet, dass** die Verbindung K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.:6), K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO.: 17), K024H901 (SEQ ID NO.: 18), K024H903 (SEQ ID NO.: 19) oder SEQ ID NO.: 20 bis SEQ ID NO.: 38 ist.

2. Verbindung nach Anspruch 1, wobei die Verbindung K024H107 (SEQ ID NO.: 10) ist.

3. Verwendung einer Verbindung nach Anspruch 1 oder 2 zur Herstellung eines Medikaments.

4. Verwendung einer Verbindung nach Anspruch 3, wobei das Medikament für die Behandlung von Obesitas bzw. Fettleibigkeit, Dyslipidämie, Cholesterinämie, Koagulationsstörungen, Syndrom X bzw. metabolischem Syndrom, Diabetes, Hypertonie oder Ateriosklerose ist.

5. Verwendung wenigstens eines Inhibitors GRK-assoziierter Signaltransduktion (GAST) zur Herstellung eines Medikaments zur Behandlung von Diabetes-assoziierten Phänomenen, wobei der GAST-Inhibitor K024H003 (SEQ ID NO.: 2), K024H007 (SEQ ID NO.: 3), K024H101 (SEQ ID NO.: 4), K024H102 (SEQ ID NO.: 5), K024H103 (SEQ ID NO.: 6), K024H104 (SEQ ID NO.: 7), K024H105 (SEQ ID NO.: 8), K024H106 (SEQ ID NO.: 9), K024H107 (SEQ ID NO.: 10), K024H108 (SEQ ID NO.: 11), K024H109 (SEQ ID NO.: 12), K024H110 (SEQ ID NO.: 13), K024H111 (SEQ ID NO.: 14), K024H112 (SEQ ID NO.: 15), K024H113 (SEQ ID NO.: 16), K024H114 (SEQ ID NO.: 17), K024H901 (SEQ ID NO.: 18), K024H903 (SEQ ID NO.: 19) oder SEQ ID NO.: 20 bis SEQ ID NO.: 38 ist.

6. Verwendung nach Anspruch 4, wobei der GAST-Inhibitor K024H107 (SEQ ID NO.: 10) ist.

7. Verwendung nach Anspruch 4 oder Anspruch 5, wobei das Diabetes-assoziierte Phänomen Diabetes Typ II, Diabetes-assoziierte Obesität bzw. Fettleibigkeit, durch Diabetes verursachte Hypertonie oder Diabetes-assoziierte Dyslipidämie ist.

## Revendications

1. Composé **caractérisé en ce que** le composé est K024H003 (SEQ ID N°2), K024H007 (SEQ ID N°3), K024H101 (SEQ ID N°4), K024H102 (SEQ ID N°5), K024H1003 (SEQ ID N°6), K024H104 (SEQ ID N°7), K024H105 (SEQ ID N°8), K024H106 (SEQ ID N°9), K024H107 (SEQ ID N°10), K024H108 (SEQ ID N°11), K024H109 (SEQ ID N°12), K024H110 (SEQ ID N°13), K024H111 (SEQ ID N°14), K024H112 (SEQ ID N°15), K024H113 (SEQ ID N°16), K024Hl14 (SEQ ID N°17), K024H901 (SEQ ID N°18), K024H903 (SEQ ID N°19) ou SEQ ID N°20 ou SEQ ID N°38.

2. Composé selon la revendication 1, dans lequel le composé est K024H107 (SEQ ID N°10).

3. Utilisation d'un composé selon la revendication 1 ou 2, pour la préparation d'un médicament.

4. Utilisation d'un composé selon la revendication 3, dans laquelle le médicament est destiné au traitement de l'obésité, de la dyslipidémie, de la cholestérolémie, des désordres de la coagulation, du syndrome X, du diabète, de l'hypertension ou de l'artériosclérose.

5. Utilisation d'au moins un inhibiteur de la transduction du signal associé au GRK (GAST) pour la préparation d'un médicament pour le traitement d'un phénomène associé au diabète, où l'inhibiteur GAST est K024H003 (SEQ ID N°2), K024H007 (SEQ ID N°3), K024H101 (SEQ ID N°4), K024H102 (SEQ ID N°5), K024H1003 (SEQ ID N°6), K024H104 (SEQ ID N°7), K024H105 (SEQ ID N°8), K024H106 (SEQ ID N°9), K024H107 (SEQ ID N°10), K024H108 (SEQ ID N°11), K024H109 (SEQ ID N°12), K024H110 (SEQ ID N°13), K024H111 (SEQ ID N°14), K024H112 (SEQ ID N°15), K024H113 (SEQ ID N°16), K024H114 (SEQ ID N°17), K024H901 (SEQ ID N°18), K024H903 (SEQ ID N°19) ou SEQ ID N°20 ou SEQ ID N°38.

6. Utilisation selon la revendication 4, dans laquelle l'inhibiteur GAST est K024H107 (SEQ ID N°10).

7. Utilisation selon la revendication 4 ou 5, dans laquelle le phénomène associé au diabète est le diabète de type II, l'obésité associée au diabète, l'hypertension associée au diabète ou la dyslipidémie associée au diabète.


### SEQUENCE LISTING

<110> Ben-Sasson, Shmuel
<120> MODULATORS OF ACTIVITY OF
   G-PROTEIN-COUPLED RECEPTOR KINASES
<130> 1242.1015-010
<140> 09/735,274
   <141> 2000-12-11
<150> PCT/US98/10319
   <151> 1998-05-20
<150> US 08/861,338
   <151> 1997-05-21
<160> 40
<170> FastSEQ for Windows Version 4.0
<210> 1
   <211> 6
   <212> PRT
   <213> Artificial Sequence
<220>
   <223> bARK1
<221> ACETYLATION
   <222> (1)... (0)
<221> AMIDATION
   <222> (0)...(6)
<400> 1
   Leu Leu Arg Gly His Ser
   1 5
<210> 2
   <211> 6
   <212> PRT
   <213> Artificial Sequence
<220>
   <223> bARK1
<221> ACETYLATION
   <222> (1) ..... (0)
   <223> D-isomer at position 4
<221> AMIDATION
   <222> (0) ... (6)
<400> 2
   Leu Leu Arg Lys His Ser
   1 5
<210> 3
   <211> 6
   <212> PRT
   <213> Artificial Sequence
<220>
   <223> bARK1
<221> ACETYLATION
   <222> (1) ... (0)
   <223> D-isomer at position 4
<221> AMIDATION
   <222> (0) ... (6)
<400> 3
   Leu Leu Arg Arg His Ser 1 5
<210> 4
   <211> 6
   <212> PRT
   <213> Artificial Sequence
<220>
   <223> bARK1
<221> MYRISTATE
   <222> (1)... (0)
<221> AMIDATION
   <222> (0) ..... (6)
<400> 4
   Leu Leu Arg Gly His Ser 1 5
<210> 5
   <211> 9
   <212> PRT
   <213> Artificial Sequence
<220>
   <223> bARK1
<221> MYRISTATE
   <222> (1) ... (0)
<221> AMIDATION
   <222> (0) ..... (9)
<400> 5
   Leu Leu Arg Gly His Ser Pro Phe Arg
   1 5
<210> 6
   <211> 6
   <212> PRT
   <213> Artificial Sequence
<220>
   <223> bARK1
<221> MYRISTATE
   <222> (1)...(0)
   <223> D-isomer at position 4
<221> AMIDATION
   <222> (0) ... (6)
<400> 6
   Leu Leu Arg Lys His Ser 1 5
<210> 7
   <211> 6
   <212> PRT
   <213> Artificial Sequence
<220>
   <223> bARK1
<221> MYRISTATE
   <222> (1)...(0)
   <223> D-isomer at position 4
<221> AMIDATION
   <222> (0) ... (6)
<400> 7
   Leu Leu Arg Glu His Ser
   1 5
<210> 8
   <211> 6
   <212> PRT
   <213> Artificial Sequence
<220>
   <223> bARK1
<221> MYRISTATE
   <222> (1) ... (0)
<221> AMIDATION
   <222> (0)...(6)
<400> 8
   Leu Leu Arg Tyr His Ser
   1 5
<210> 9
   <211> 6
   <212> PRT
   <213> Artificial Sequence
<220>
   <223> bARK1
<221> MYRISTATE
   <222> (1) ... (0)
   <223> D-isomer a position 4
<221> AMIDATION
   <222> (0)...(6)
<400> 9
   Leu Leu Arg Leu His Ser
   1 5
<210> 10
   <211> 6
   <212> PRT
   <213> Artificial Sequence
<220>
   <223> bARK1
<221> MYRISTATE
   <222> (1)...(0)
   <223> D-isomer at position 4
<221> AMIDATION
   <222> (0) ..... (6)
<400> 10
   Leu Leu Arg Arg His Ser
   1 5
<210> 11
   <211> 7
   <212> PRT
   <213> Artificial Sequence
<220>
   <223> bARK1
<221> MYRISTATE
   <222> (1)...(0)
   <223> D-isomer at position 4
   Position 7 is biotinylated
<221> AMIDATION
   <222> (0)...(7)
<400> 11
   Leu Leu Arg Arg His Ser Lys
   1 5
<210> 12
   <211> 7
   <212> PRT
   <213> Artificial Sequence
<220>
   <223> bARK1
<221> MYRISTATE
   <222> (1)...(0)
   <223> D-isomer at position 4
<221> AMIDATION
   <222> (0) ... (7)
<400> 12
   Leu Leu Arg Lys His Ser Pro
   1 5
<210> 13
   <211> 9
   <212> PRT
   <213> Artificial Sequence
<220>
   <223> bARK1
<221> MYRISTATE
   <222> (1) ... (0)
   <223> D-isomer at position 4
<221> AMIDATION
   <222> (0)...(9)
<400> 13
   Leu Leu Arg Arg His Ser Ile Val Thr
    1               5
<210> 14
   <211> 8
   <212> PRT
   <213> Artificial Sequence
<220>
   <223> BARKI
<221> MYRISTATE
   <222> (1)...(0)
   <223> D-isomer at position 4
<221> AMIDATION
   <222> (0)...(8)
<400> 14
   Leu Leu Arg Arg His Ser Ile Val
    1               5
<210> 15
   <211> 7
   <212> PRT
   <213> Artificial Sequence
<220>
   <223> bARK1
<221> MYRISTATE
   <222> (1) ... (0)
   <223> D-isomer at position 4
<221> AMIDATION
   <222> (0) ... (7)
<400> 15
   Leu Leu Arg Arg His Ser Ile
    1               5
<210> 16
   <211> 7
   <212> PRT
   <213> Artificial Sequence
<220>
   <223> bARK1
<221> MYRISTATE
   <222> (1) ... (0)
   <223> D-isomer at position 4 Benzoylamide at position 7
<221> AMIDATION
   <222> (0) ... (7)
<400> 16
   Leu Leu Arg Arg His Ser Lys
   1 5
<210> 17
   <211> 7
   <212> PRT
   <213> Artificial Sequence
<220>
   <223> bARK1
   Oleyl at position 1
   D-isomer at position 4
   Benzoylamide at position 7
<221> AMIDATION
   <222> (0)...(7)
<400> 17
   Leu Leu Arg Arg His Ser Lys
   1 5
<210> 18
   <211> 6
   <212> PRT
   <213> Artificial Sequence
<220>
   <223> bARK1
   Strearate at position 1
<221> AMIDATION
   <222> (0) ... (6)
<400> 18
   Leu Leu Arg Gly His Ser
   1 5
<210> 19
   <211> 6
   <212> PRT
   <213> Artificial Sequence
<220>
   <223> bARK1
   Stearate at position 1
   D-isomer at position 4
<221> AMIDATION
   <222> (0) ... (6)
<400> 19
   Leu Leu Arg Lys His Ser
   1               5
<210> 20
   <211> 32
   <212> PRT
   <213> Artificial Sequence
<400> 20
   Phe Lys Leu Leu Arg Gly His Ser Pro Phe Arg Gln His Lys Thr Asp
   1               5                   10                  15
   Lys His Glu Ile Asp Arg Met Thr Leu Thr Met Ala Val Glu Leu Pro
               20                  25                  30
<210> 21
   <211> 32
   <212> PRT
   <213> Artificial Sequence
<400> 21
   Phe Lys Leu Leu Arg Gly His Ser Pro Phe Arg Gln His Lys Thr Asp
   1               5                   10                  15
   Lys His Glu Ile Asp Arg Met Thr Leu Thr Val Asn Val Glu Leu Pro
               20                  25                  30
<210> 22
   <211> 20
   <212> PRT
   <213> Artificial Sequence
<220>
   <223> GRK2
<400> 22
   Leu Asp Leu Met Asn Gly Gly Asp Leu His Tyr His Leu Ser Gln His
   1 5 10 15
   Gly Val Phe Ser
   20
<210> 23
   <211> 20
   <212> PRT
   <213> Artificial Sequence
<220>
   <223> GRK3
<400> 23
   Leu Asp Leu Met Asn Gly Gly Asp Leu His Tyr His Leu Ser Gln His
   1 5 10 15
   Gly Val Phe Ser
   20
<210> 24
   <211> 9
   <212> PRT
   <213> Artificial Sequence
<220>
   <223> GRK2
<400> 24
   Met Ser Tyr Ala Phe His Thr Pro Asp
   1 5
<210> 25
   <211> 9
   <212> PRT
   <213> Artificial Sequence
<220>
   <223> GRK3
<400> 25
   Met Thr Tyr Ala Phe His Thr Pro Asp
   1 5
<210> 26
   <211> 21
   <212> PRT
   <213> Artificial Sequence
<220>
   <223> GRK2
<400> 26
   Arg Ile Met Leu Ser Leu Val Ser Thr Gly Asp Cys Pro Phe Ile Val
   1 5 10 15
   Cys Met Ser Tyr Ala
   20
<210> 27
   <211> 21
   <212> PRT
   <213> Artificial Sequence
<220>
   <223> GRK3
<400> 27
   Arg Ile Met Leu Ser Leu Val Ser Thr Gly Asp Cys Pro Phe Ile Val
   1 5 10 15
   Cys Met Thr Tyr Ala
   20
<210> 28
   <211> 11
   <212> PRT
   <213> Artificial Sequence
<220>
   <223> Myristylistyl at position 1
<400> 28
   Gly Gly Arg Gly His Ser Pro Phe Arg Gln His
   1 5 10
<210> 29
   <211> 10
   <212> PRT
   <213> Artificial Sequence
<220>
   <223> Myristylistyl at position 1
<400> 29
   Gly Gly Arg Gly His Ser Pro Phe Arg Gln
   1 5 10
<210> 30
   <211> 9
   <212> PRT
   <213> Artificial Sequence
<220>
   <223> Myristylistyl at position 1
<400> 30
   Gly Gly Arg Gly His Ser Pro Phe Arg
   1 5
<210> 31
   <211> 9
   <212> PRT
   <213> Artificial Sequence
<220>
   <223> Myristylistyl at position 1
<400> 31
   Gly Gly His Ser Pro Phe Arg Gln His
   1 5
<210> 32
   <211> 8
   <212> PRT
   <213> Artificial Sequence
<220>
   <223> Myristylistyl at position 1
<400> 32
   Gly Gly His Ser Pro Phe Arg Gln
   1 5
<210> 33
   <211> 9
   <212> PRT
   <213> Artificial Sequence
<220>
   <223> Myristylistyl at position 1
<400> 33
   Gly Gly Pro Phe Arg Gln His Lys Thr
   1 5
<210> 34
   <211> 9
   <212> PRT
   <213> Artificial Sequence
<220>
   <223> Myristylistyl at position 1
<400> 34
   Gly Gly Phe Arg Gln His Lys Thr Lys
   1 5
<210> 35
   <211> 8
   <212> PRT
   <213> Artificial Sequence
<220>
   <223> Myristylistyl at position 1
<400> 35
   Gly Leu Leu Arg His Arg Ser Ile
   1 5
<210> 36
   <211> 8
   <212> PRT
   <213> Artificial Sequence
<220>
   <223> Myristylistyl at position 1
<400> 36
   Gly Leu Leu Arg His Arg Ala Ala
   1 5
<210> 37
   <211> 8
   <212> PRT
   <213> Artificial Sequence
<220>
   <223> Myristylistyl at position 1
<400> 37
   Gly Leu Leu Arg Arg His Ser Ile
   1 5
<210> 38
   <211> 8
   <212> PRT
   <213> Artificial Sequence
<220>
   <223> Lauryl at position 1
<400> 38
   Gly Leu Leu Arg Arg His Ser Ile
   1 5
<210> 39
   <211> 30
   <212> DNA
   <213> Artificial Sequence
<220>
   <223> GRK2
<400> 39
   ctcggcctcg ggcgcggccg agcgccgcgc 30
<210> 40
   <211> 30
   <212> DNA
   <213> Artificial Sequence
<220>
   <223> GRK3
<400> 40
   caagcttcat ctgtatttac agctgctcgc 30